# EUROPEAN PATENT SPECIFICATION

(11) **EP 2 043 630 B1**
(45) Date of publication and mention of the grant of the patent: **07.09.2016**
(21) Application number: 07799439.0
(22) Date of filing: 10.07.2007
(51) Int. Cl.: A61K 8/49, A61Q 19/08, A61K 9/00

(54) **6, 9-DISUBSTITUTED PURINE DERIVATIVES FOR COSMETIC USE**
6, 9-DISUBSTITUIERTE PURINDERIVATE ZUR KOSMETISCHEN ANWENDUNG
DÉRIVÉS DE PURINE 6,9-DISUBSTITUÉS À USAGE COSMÉTIQUE

(30) Priority: 10.07.2006 US 806871 P; 09.07.2007 US 774652
(43) Date of publication of application: 08.04.2009
(73) Proprietor: Institute of Experimental Botany, Academy of Sciences of the Czech Republic, 165 02 Prague 6 - Lysolaje (CZ)
(72) Inventor: SZUCOVA, Lucie, Nove Sady 779 00 (CZ); ZATLOUKAL, Marek, 787 01 Sumperk (CZ); SPICHAL, Lukas, 772 00 Olomouc (CZ); FROHLICH, Ludek, 779 00 Olomouc (CZ); DOLEZAL, Karel, 779 00 Olomouc (CZ); STRNAD, Miroslav, 779 00 Olomouc (CZ); MASSINO, Frank J., Napa, California 94558 (US)
(74) Representative: Hoffmann Eitle
(86) International application number: PCT/US2007/073142
(87) International publication number: WO 2008/008770

(56) References cited:
- WO-A2-2004/037159
- WO-A2-2007/007255
- US-A- 5 021 422
- US-A- 5 602 139
- US-A- 5 602 139
- US-A1- 2005 113 340
- US-A1- 2005 113 340
- ROLAND K ROBINS ET AL: "Purine Nucleosides. I. The Synthesis of Certain 6-Substituted-9-(tetrahydro-2- pyrany1)-purines as Models of Purine Deoxynucleosidesl", JOURNAL OF THE AMERICAN CHEMICAL SOCIETY, AMERICAN CHEMICAL SOCIETY, US, vol. 83, 5 June 1961 (1961-06-05), pages 2574-2579, XP008129304, ISSN: 0002-7863

## Description

### Related Application

### Technical Field

The present invention provides the use of compounds of Formula (I) for improving the cosmetic appearance of mammalian epidermal cells or human skin cells.

The present application is defined in the appended claims. Subject matters which are not encompassed by the scope of the claims do not form part of the present invention.

### Background

Cellular aging or cellular senescence is a universal attribute of normal non-transformed cells that is manifested by morphological changes accompanied by an age-dependent loss of proliferative potential or capacity, including the failure of the cells to respond to exogenous growth factors. A variety of theories have been proposed to explain the phenomenon of cellular senescence. Experimental evidence suggests that the age-dependent loss of proliferative potential or capacity may be the function of a genetic program (see, e.g., Smith et al., Mech. Age. Dev. 13, 387 (1980); and Kirkwood et al., Theor. Biol. 53, 481 (1975)). This evidence includes cell fusion studies with human fibroblasts *in vitro* that demonstrate that the quiescent cellular senescent phenotype is dominant over the proliferative phenotype (see, e.g., Pereira-Smith et al., Somatic Cell Genet. 8, 731 (1982); and Norwood et al., Proc. Natl. Acad. Sci. USA 1, 223 (1974)) and that protein synthesis in senescent cells, prior to fusion with young cells, is required for the inhibition of DNA synthesis within the young nucleus of the heterodikaryon (see, e.g., Burmer et al., Exp. Cell Res. 145, 708 (1983); and Drescher-Lincoln et al., Exp. Cell Res. 153, 208 (1984)). Also, microinjection of senescent fibroblast mRNA into young fibroblasts inhibits the ability of the young cell to synthesize DNA (see, e.g., Lumpkin et al., Science 232, 393 (1986)) and entry of the young cell into the S phase of the cell cycle (Lumpkin et al., Exp. Cell Res. 160, 544 (1985)). Further, unique mRNA species are amplified in senescent fibroblasts *in vitro* (see, e.g., Wellinger et al., J. Cell Biol., 34, 203 (1986); Flemming et al., Proc. Natl. Acad. Sci. USA 85, 4099 (1988); West et al., Exp. Cell Res. 184, 138 (1989); and Giordano, Exp. Cell Res. 185, 399 (1989)). It has also been suggested that an altered genetic program exists in senescent human fibroblasts, which involves the repression of c-fos expression at the transcriptional level (see, e.g., Seshadri et al., Science 247, 205 (1990)). Thus, there appear to be genotypic, as well as phenotypic, differences between young and old cells.

In recent years, 6-substituted aminopurines have assumed considerable biochemical significance. Some compounds of this type promote plant growth and belong to the group of growth regulators termed "cytokinins" (Letham, Ann. Rev. Plant. Physiol. 18, 349 (1967)). In bioassays based on induction of cell division in plant tissue cultures, the most active cytokinin compound is the naturally occurring cytokinin *trans*-zeatin (6-((E)-4-hydroxy-3-methylbut-2-enylamino)purine, Letham, Planta 74, 228 (1967)). Cytokinins closely related to zeatin occur as bases in soluble RNA (Skoog et al., Science 154, 1354 (1966)). In the serine and tyrosine RNAs of yeast, plants and animals the cytokinin is adjacent to the anticodon. The growth of mammalian cell cultures is inhibited by certain N⁶-substituted adenosines with cytokinin activity (Grace et al., Proc. Am. Assoc. Cancer Res. 8, 23 (1967)). With stem segments, leaf cuttings and developing grapes, 6-benzylamino-9-(2-tetrahydropyranyl)purine (BPA) had been reported to evoke greater growth than the cytokinin 6-benzylaminopurine (BA). In tissue culture bioassays and some horticulture species, BPA has also been shown to be more active (Werbrouck et al., Physiol. Plant 98, 291 (1996)).

Additionally, certain 6-(substituted amino)purines (including kinetin and zeatin) have been shown to have significant anti-aging and other properties and have been found useful for the treatment of mammalian cells, including human skin and/or human skin cells. Topically application of such compositions allowed for improvement of cosmetic appearance of skin; such compositions could also be used for treatment of skin and related diseases or conditions. Importantly, these 6-(substituted amino)purines do not, in the amounts used, substantially increase the growth rate and total proliferative capacity of the treated mammalian cells. See, e.g., U.S. Patents 5,371,089 (December 6, 1994) and 5,602,139 (February 11, 1997) (improvement of cosmetic appearance of skin); U.S. Patent 5,614,407 (March 25, 1997) (slow or delay morphological changes that normally accompany aging of mammalian cells in cultures); and U.S. Patents 5,021,422 (June 4, 1991) and 5,164,394 (November 17, 1992) (treatment of certain hyperproliferative skin diseases). All of the just-listed patents are hereby incorporated by reference. The antiaging actions of 6-(substituted amino)purines on human skin were also demonstrated by Rattan and Clark (Biochem. Biophys. Res. Commun. 201, 665-672 (1994)) in cultured human fibroblasts, where the presence of kinetin (N6-furfuryl-adenine) delayed the onset and decreased the extent of many morphological and biochemical characteristics associated with serial passaging of cells. The effectiveness of such 6-(substituted amino)purines in maintaining normal cell function in aging cells provides the basis for their use in preserving the vitality of aging skin. More recently, clinical studies conducted at the University of California, Irvine by Dr. Gerald Weinstein (Cosmetic Dermatology 15, 29-32 (2003)) showed that topical kinetin products ranging in concentration from 0.005 to 0.10% (Kinerase^{®}) improved the appearance of mild to moderately photodamaged facial skin. Treatments after 12 and 24 weeks produced significant improvement in the appearance of skin texture, mottled hyperpigmentation, and fine wrinkles compared to baseline as assessed by both the physician and the subjects. Treatments also produced an improvement in skin barrier function as assessed by a decrease in transepidermal water loss.

Preventing, reversing, or slowing the process of cellular aging has been a persistent, though elusive, goal of biological science, that would have a number of significant and practical consequences. Preventing the aging of cells in human skin or other organs would be associated with preservation of structural and functional integrity and also cosmetic integrity. If cultured cells could be treated so that they retain characteristics of young cells, the production of valuable products by such cells in culture could be improved.

Although 6-(substituted amino)purines, especially kinetin and zeatin, have significant anti-aging and other properties, it would be desirable to provide additional growth-regulatory, differentiating, and/or anti-senescent compounds. It would be especially desirable if such compounds have improved selectivity and efficiency (i.e., less toxic and/or more efficacious) than currently used 6-(substituted amino)purines. The present invention provides such improved anti-aging compounds.

### Summary of the Invention

The present invention is directed to 6,9-disubstituted purine derivatives of the general formula 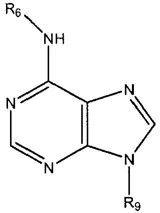 and pharmaceutically acceptable salts thereof, wherein R₆ is furfuryl, methoxy-substituted furfuryl, phenyl, methoxy-substituted phenyl, and methoxy-substituted benzyl; and wherein R₉ is 2-tetrahydropyranyl or 2-tetrahydrofuranyl. Methoxy-substituted furfuryl groups include, for example, 3-methoxyfurfuryl, 4-methoxyfurfuryl, and 5-methoxyfurfuryl. Methoxy-substituted phenyl groups include, for example, 2-methoxyphenyl, 3-methoxyphenyl, 4-methoxyphenyl, 2,3-dimethoxyphenyl, 2,4-dimethoxyphenyl, 2,5-dimethoxylphenyl, 3,4-dimethoxylphenyl, 3,5-dimethoxylphenyl, 2,3,4-trimethoxyphenyl, and 2,3,5-trimethoxyphenyl. Methoxy-substituted benzyl groups include, for example, 2-methoxybenzyl, 3-methoxybenzyl, 4-methoxybenzyl, 2,3-dimethoxybenzyl, 2,4-dimethoxybenzyl, 2,5-dimethoxybenzyl, 3,4-dimethoxybenzyl, 3,5-dimethoxybenzyl 2,3,4-trimethoxybenzyl, and 2,3,5-trimethoxybenzyl. The preferred 6,9-disubstituted purine derivative is 6-furfurylamino-9-(2-tetrahydropyranyl)purine (also referred to as N6-furfuryl-9-(2-tetrahydropyranyl)adenine or pyranyl kinetin) which has the general formula 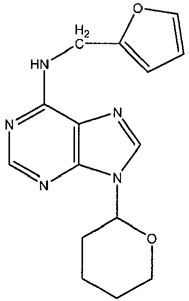 These 6,9-disubstituted purine derivatives have been found to possess anti-senescent, anti-inflammatory, and/or immunosuppressive properties.

The 6,9-disubstituted purine derivatives of of the present claims are useful as cosmetic compositions for inhibiting aging and senescence, improving the cosmetic appearance of mammalian epidermal cells, such as keratinocytes or fibroblasts, and/or ameliorating the adverse effect of aging in mammalian epidermal cells, such as keratinocytes or fibroblasts. They are especially useful as cosmetic compositions for inhibiting aging and senescence and/or improving the cosmetic appearance of human epidermal cells and/or human skin. Thus, the present invention provides a method for ameliorating the adverse effect of aging in mammalian cells, said method comprising applying an effective amount of a 6,9-disubstituted purine derivative to the mammalian cells, wherein the 6,9-disubstituted purine derivative is of the general formula 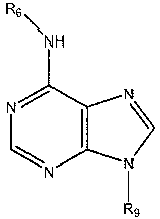 or a pharmaceutically acceptable salt thereof, wherein R₆ is furfuryl, methoxy-substituted furfuryl, phenyl, methoxy-substituted phenyl, and methoxy-substituted benzyl; and wherein R₉ is 2-tetrahydropyranyl or 2-tetrahydrofuranyl.

In some aspects, the 6,9-disubstituted purine derivatives are also useful for treatment of certain skin diseases, including hyperproliferative skin diseases. Compositions containing these 6,9-disubstituted purine derivatives can, for example, be used for treatment of skin conditions such as lupus, allergic eczema, toxic eczema, atopic dermatitis, ichtyosis, papilloma, Bowen's disease, seborrhoic keratosis, actinic keratosis, basal and squamous cell carcinoma, and the like. Thus, the disclosure also provides a method for treating skin diseases in mammalian cells, said method comprising applying an effective amount of a 6,9-disubstituted purine derivative to the mammalian cells needing such treatment, wherein the 6,9-disubstituted purine derivative is of the general formula 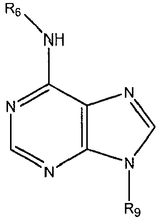 or a pharmaceutically acceptable salt thereof, wherein R₆ is furfuryl, methoxy-substituted furfuryl, phenyl, methoxy-substituted phenyl, and methoxy-substituted benzyl; and wherein R₉ is 2-tetrahydropyranyl or 2-tetrahydrofuranyl.

In some aspects, the 6,9-disubstituted purine derivatives are also useful for treating inflammation-related conditions. Such inflammation-related conditions include, for example, inflammation, lesions (e.g., accelerating healing thereof), pain and other immunological responses resulting from inflammation (e.g., providing relief thereof), and/or treating inflammation skin diseases (e.g., atopic dermatitis, lichen planus, hyperpigmentation, Herpes simplex lesions, and the like). Thus, the disclosure also provides a method for treating inflammation conditions in mammalian cells, said method comprising applying an effective amount of a 6,9-disubstituted purine derivative to the mammalian cells needing such treatment, wherein the 6,9-disubstituted purine derivative is of the general formula 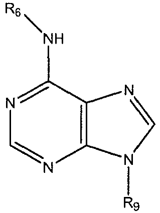 or a pharmaceutically acceptable salt thereof, wherein R₆ is furfuryl, methoxy-substituted furfuryl, phenyl, methoxy-substituted phenyl, and methoxy-substituted benzyl; and wherein R₉ is 2-tetrahydropyranyl or 2-tetrahydrofuranyl.

The 6,9-disubstituted purine derivatives of this invention may be used in compositions in the form of free compounds of the above formulae or as pharmaceutically acceptable salts thereof. Pharmaceutically acceptable salts may be formed with, for example, alkali metals, ammonium, or amines. The derivatives or their salts may be in the form of a racemate mixture or optically active isomers; they may also be in the form of addition salts with acids.

### Detailed Description

The 6,9-disubstituted purine derivatives of this invention have the general formula 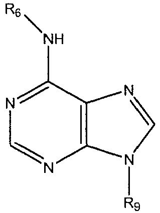 wherein R₆ is furfuryl, methoxy-substituted furfuryl, phenyl, methoxy-substituted phenyl, and methoxy-substituted benzyl; and wherein R₉ is 2-tetrahydropyranyl or 2-tetrahydrofuranyl. Methoxy-substituted furfuryl groups include, for example, 3-methoxyfurfuryl, 4-methoxyfurfuryl, and 5-methoxyfurfuryl. Methoxy-substituted phenyl groups include, for example, 2-methoxyphenyl, 3-methoxyphenyl, 4-methoxyphenyl, 2,3-dimethoxyphenyl, 2,4-dimethoxyphenyl, 2,5-dimethoxylphenyl, 3,4-dimethoxyphenyl, 3,5-dimethoxylphenyl, 2,3,4-trimethoxyphenyl, and 2,3,5-trimethoxyphenyl. Methoxy-substituted benzyl groups include, for example, 2-methoxybenzyl, 3-methoxybenzyl, 4-methoxybenzyl, 2,3-dimethoxybenzyl, 2,4-dimethoxybenzyl, 2,5-dimethoxybenzyl, 3,4-dimethoxybenzyl, 3,5-dimethoxybenzyl, 2,3,4-trimethoxybenzyl, and 2,3,5-trimethoxybenzyl. The preferred 6,9-disubstituted purine derivative is 6-furfurylamino-9-(2-tetrahydropyranyl)purine. These 6,9-disubstituted purine derivatives have been found to possess anti-senescent, anti-inflammatory, and/or immunosuppressive properties when contacted with mammalian cells, including human cells.

The 6,9-disubstituted purine derivatives of this invention are useful as cosmetic compositions for inhibiting aging and senescence and/or improving the cosmetic appearance of mammalian epidermal cells, such as keratinocytes or fibroblasts. They are especially useful as cosmetic compositions for inhibiting aging and senescence and/or improving the cosmetic appearance of human epidermal cells and/or human skin.

In some aspects, the 6,9-disubstituted purine derivatives are also useful for treatment of certain skin diseases or conditions, including (but not limited to) hyperproliferative skin diseases. Compositions containing these 6,9-disubstituted purine derivatives can, for example, be used for treatment of lupus, allergic eczema, toxic eczema, atopic dermatitis, ichtyosis, papilloma, Bowen's disease, seborrhoic keratosis, actinic keratosis, basal and squamous cell carcinoma, acne, erythema, and the like.

In some aspects, the 6,9-disubstituted purine derivatives are also useful for treating inflammation, accelerating healing of lesions, providing relief of pain and other immunological responses resulting from inflammation, and/or treating inflammation skin diseases or conditions (e.g., atopic dermatitis, lichen planus, hyperpigmentation, Herpes simplex lesions, erythema, and the like).

The 6,9-disubstituted purine derivatives of this invention may be used in compositions in the form of free compounds of the above formulae or as pharmaceutically acceptable salts thereof; a single or a mixture of such 6,9-disubstituted purine derivatives may be used. Pharmaceutically acceptable salts may be formed with, for example, alkali metals, ammonium, or amines. The derivatives or their salts may be in the form of a racemate mixture or optically active isomers; they may also be in the form of addition salts with acids.

The 6,9-disubstituted purine derivatives of this invention are generally contained in a carrier composition suitable for application to the cells of interest (e.g., human skin) and in an amount suitable to the intended use. Such compositions include, for example, cosmetic and pharmaceutical compositions. Generally, such compositions comprise about 0.005 to about 20 percent of the active ingredient, preferably about 0.05 to about 10 percent, and more preferably about 0.1 to about 2 percent. The compositions, especially the cosmetic and pharmaceutical compositions, can be in the form of solutions, creams, aerosols, milky lotions, lotions, gels, plasters, poultices, shampoos, lipsticks, ointments, pastes, foams, tinctures, sprays, and the like. The form of the composition is not critical as long as it is suitable for its intended use.

Cosmetic and pharmaceutical carriers or vehicles suitable for administration of the compounds provided herein include any such carriers known to those skilled in the art to be suitable for the particular mode of administration. In addition, the compounds may be formulated as the sole pharmaceutically active ingredient in the composition or may be combined with other active ingredients. The active compound is included in the carrier in an amount sufficient to exert a therapeutically and/or cosmetically useful effect in the absence of serious toxic effects on the treated individual. The effective concentration may be determined empirically by testing the compounds using *in vitro* and *in vivo* systems, including tissue culture and hairless mice or other suitable animal models. Therapeutically and/or cosmetically useful effects include, but are not limited to, delaying, slowing, preventing, reversing, reducing, and otherwise modifying in a beneficial manner, a disease state or adverse cosmetic effects associated with aging of mammalian cells, especially human cells, and even more especially human skin cells. Such cosmetic effects can include, for example, improving the appearance of human skin already damaged by aging or sun/wind exposure, preventing (or slowing) the occurrence of such damage in the first place in undamaged skin, and/or preventing (or slowing) the occurrence of additional such damage in skin already damaged. Such therapeutic effects can include, for example, improving the condition of human skin already damaged by a disease state, preventing (or slowing) the occurrence of such a disease state in the first place, and/or preventing (or slowing) the reoccurrence of additional such disease states.

The concentration of active compound in the composition will depend on absorption, inactivation, excretion rates of the active compound, the dosage schedule, and amount administered as well as other factors known to those of skill in the art. Typically a therapeutically and/or cosmetically effective dosage should deliver a concentration of at least about 0.005 percent, preferably at least about 0.05, and more preferably at least about 0.1 percent of the active compound to the treated tissue. The active ingredient may be administered at once, or may be divided into a number of smaller doses to be administered at intervals of time. It is understood that the precise dosage and duration of treatment is a function of the tissue being treated and may be determined empirically using known testing protocols or by extrapolation from *in vivo* or *in vitro* test data. It is to be noted that concentrations and dosage values may also vary with the age of the individual treated. It is to be further understood that for any particular subject, specific dosage regimens should be adjusted overtime according to the individual need and the professional judgment of the person administering or supervising the administration of the compositions, and that the concentration ranges set forth herein are exemplary only and are not intended to limit the scope or practice of the claimed compositions.

For treatment of the skin, the compounds may be formulated as cosmetic or pharmaceutical compositions for local or topical application to the skin in which the 6,9-disubstituted purine derivatives are mixed with a pharmaceutically or cosmetically acceptable carrier. The compositions may be provided in the form of gels, creams, lotions, solids, solutions, suspensions, aerosols, and the like. Compositions for treating human skin are formulated for topical application with a 6,9-disubstituted purine derivative in an effective concentration range, between about 0.005 to about 20 percent, preferably about 0.05 to about 10 percent, and more preferably about 0.1 to about 2 percent in a cream, ointment, lotion, gel, solution, solid base or vehicle known in the art to be non-toxic and dermatologically acceptable. The concentration or weight fraction of the 6,9-disubstituted purine derivative dissolved, suspended, dispersed, or otherwise mixed in a composition for use with human skin will be such that the 6,9-disubstituted purine derivative is delivered at an effective concentration, generally at least about 0.005 percent, preferably at least about 0.05 percent, and preferably at least about 0.1 percent to active cells of the skin (e.g., fibroblasts) such that the adverse effects of aging are reduced, reversed, or delayed. The upper limit should be adjusted such that the rate of cell division or total proliferative capacity of the cells is not substantially increased, particularly such that the treated cells or tissues do not exhibit any signs typical of cancerous or pre-cancerous alterations or any other cosmetically undesirable changes, such as the development of lesions. Although the upper limit can be up to about 20 percent, lower upper limits (such as 10 percent, 2 percent, or even 1 percent) are generally preferred since the anti-aging effects are evident at such lower rates and the risk of undesirable increase in the rate of cell division or total proliferative capacity of the cells is minimized. Generally, emollient or lubricating vehicles that help hydrate the skin are more preferred than volatile vehicles, such as ethanol, that dry the skin.

Examples of suitable bases or vehicles for preparing compositions for use with human skin are petrolatum, petrolatum plus volatile silicones, lanolin, cold cream (USP), and hydrophilic ointment (USP). Compositions can be prepared containing an effective amount of one or more 6,9-disubstituted purine derivatives formulated for topical application, such as emulsified, suspended or otherwise mixed with a suitable ointment or cream base.

The choice of an acceptable vehicle is largely determined by the way the 6,9-disubstituted purine derivative is to be administered. Such methods include topical administration. Suitable pharmaceutically and dermatologically acceptable vehicles for topical application include those suited for use in lotions, creams, solutions, suspensions, gels, solids and the like. Generally, the vehicle is either organic in nature or an aqueous emulsion and capable of having the 6,9-disubstituted purine derivative dispersed, suspended, or dissolved therein. The vehicle may include, for example, pharmaceutically-acceptable emollients, skin absorption enhancers, UV protectants, anti-oxidants, buffers, coloring agents, fragrances, emulsifiers, fillers, thickening agents, solvents, and the like.

A more detailed list and description of such forms (which is not intended to be exhaustive) follows:
(1) Lotions. The lotions contain an effective concentration of one or more 6,9-disubstituted purine derivatives. The effective concentration is preferably effective to deliver the 6,9-disubstituted purine derivatives at a concentration of between about 0.05 to about 10 percent to active cells of the skin, particularly the fibroblasts in the dermis. The lotions may also contain from about 1 to about 50 percent, preferably from about 3 to about 15 percent, of an emollient and the balance water, a suitable buffer, a C₂ or C₃ alcohol, or a mixture of water or the buffer and the alcohol. Any emollients known to those of skill in the art as suitable for application to human skin may be used. These include, but are not limited to, the following:
   (a) Hydrocarbon oils and waxes including (but not limited to) mineral oil, petrolatum, paraffin, ceresin, ozokerite, microcrystalline wax, polyethylene, and perhydrosqualene.
   (b) Silicone oils including (but not limited to) dimethylpolysiloxanes, methylphenylpolysiloxanes, water-soluble and alcohol-soluble silicone-glycol copolymers.
   (c) Triglyceride fats and oils, including those derived from vegetable, animal and marine sources. Examples include (but are not limited to) castor oil, safflower oil, cotton seed oil, corn oil, olive oil, cod liver oil, almond oil, avocado oil, palm oil, sesame oil, and soybean oil.
   (d) Acetoglyceride esters including (but not limited to) acetylated monoglycerides.
   (e) Ethoxylated glycerides including (but not limited to) such as ethoxylated glyceryl monstearate.
   (f) Alkyl esters of fatty acids having 10 to 20 carbon atoms. Methyl, isopropyl and butyl esters of fatty acids are useful herein. Examples include (but are not limited to) hexyl laurate, isohexyl laurate, isohexyl palmirate, isopropyl palmirate, isopropyl myristate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
   (g) Alkenyl esters of fatty acids having 10 to 20 carbon atoms. Examples thereof include (but are not limited to) oleyl myristate, oleyl stearate, and oleyl oleate.
   (h) Fatty acids having 9 to 22 carbon atoms. Suitable examples include (but are not limited to) pelargonic, lauric, myristic, palmitic, stearic, isostearic, hydroxystearic, oleic, linoleic, ricinoleic, arachidonic, behenic, and erucic acids.
   (i) Fatty alcohols having 10 to 22 carbon atoms including (but not limited to) lauryl, myristyl, cetyl, hexadecyl, stearyl, isostearyl, hydroxystearyl, oleyl, ricinoleyl, behenyl, erucyl, and 2-octyl dodecyl alcohols.
   (j) Fatty alcohol ethers including (but not limited to) to ethoxylated fatty alcohols of 10 to 20 carbon atoms, such as (but are not limited to) the lauryl, cetyl, stearyl, isostearyl, oleyl, and cholesterol alcohols having attached thereto from 1 to 50 ethylene oxide groups or 1 to 50 propylene oxide groups or mixtures thereof.
   (k) Ether-esters including (but not limited to) fatty acid esters of ethoxylated fatty alcohols.
   (l) Lanolin and derivatives including (but not limited to) lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases.
   (m) Polyhydric alcohols and polyether derivatives including (but not limited to) propylene glycol, dipropylene glycol, polypropylene glycol (M.W. 2000-4000), polyoxyethlene polyoxypropylene glycols, polyoxypropylene polyoxyethylene glycols, glycerol, ethoxylated glycerol, propoxylated glycerol, sorbitol, ethoxylated sorbitol, hydroxypropyl sorbitol, polyethylene glycol (M.W. 200-6000), methoxy polyethylene glycols 350, 550, 750, 2000, 5000, poly[ethylene oxide] homopolymers (M.W. 100,000-5,000,000), polyalkylene glycols and derivatives, hexylene glycol (2-methyl-2,4-pentanediol), 1,3-butylene glycol, 1,2,6,-hexanetriol, ehtohexadiol USP (2-ethyl-1,3-hexanediol), C₁₅ -C₁₈ vicinal glycol and polyoxypropylene derivatives of trimethylolpropane.
   (n) Polyhydric alcohol esters including (but not limited to) ethylene glycol mono- and di-fatty acid esters, diethylene glycol mono- and di-fatty acid esters, polyethylene glycol (M.W. 200-6000), mono- and di-fatty esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.
   (o) Wax esters including (but not limited to) beeswax, spermaceti, myristyl myristate, and stearyl stearate and beeswax derivatives, including, but not limited to, polyoxyethylene sorbitol beeswax, which are reaction products of beeswax with ethoxylated sorbitol of varying ethylene oxide content that form a mixture of ether-esters.
   (p) Vegetable waxes including (but not limited to) carnauba and candelilla waxes.
   (q) Phospholipids including (but not limited to) lecithin and derivatives.
   (r) Sterols including (but not limited to) cholesterol and cholesterol fatty acid esters.
   (s) Amides including (but not limited to) fatty acid amides, ethoxylated fatty acid amides, and solid fatty acid alkanolamides.

   The lotions may also contain from about 1 to about 10 percent, more preferably from about 2 to about 5 percent, of an emulsifier. The emulsifiers can be nonionic, anionic or cationic. Examples of satisfactory nonionic emulsifiers include (but are not limited to) fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono- and di-fatty acid esters of ethylene oxide, mono- and di-fatty acid esters of ethylene glycol wherein the fatty acid moiety contains from 10 to 20 carbon atoms, diethylene glycol, polyethylene glycols of molecular weight 200 to 6000, propylene glycols of molecular weight 200 to 3000, glycerol, sorbitol, sorbitan, polyoxyethylene sorbitol, polyoxyethylene sorbitan and hydrophilic wax esters. Suitable anionic emulsifiers include (but are not limited to) the fatty acid soaps, e.g., sodium, potassium and triethanolamine soaps, wherein the fatty acid moiety contains from 10 to 20 carbon atoms. Other suitable anionic emulsifiers include (but are not limited to) the alkali metal, ammonium or substituted ammonium alkyl sulfates, alkyl arylsulfonates, and alkyl ethoxy ether sulfonates having 10 to 30 carbon atoms in the alkyl moiety. The alkyl ethoxy ether sulfonates contain from 1 to 50 ethylene oxide units. Among satisfactory cationic emulsifiers are quaternary ammonium, morpholinium and pyridinium compounds. Certain of the emollients described in preceding paragraphs also have emulsifying properties. When a lotion is formulated containing such an emollient, an additional emulsifier is not needed, though it can be included in the composition.
   The balance of the lotion is generally water or a C₂ or C₃ alcohol, or a mixture of water and the alcohol. The lotions can be formulated by simply admixing all of the components together. Preferably the 6,9-disubstituted purine derivative is dissolved, suspended or otherwise uniformly dispersed in the mixture.
   Other conventional components of such lotions may be included. One such additive is a thickening agent at a level from about 1 to about 10 percent of the composition. Examples of suitable thickening agents include, but are not limited to, cross-linked carboxypolymethylene polymers, ethyl cellulose, polyethylene glycols, gum tragacanth, gum kharaya, xanthan gums and bentonire, hydroxyethyl cellulose, and hydroxypropyl cellulose.
   One preferred example of a composition suitable for application to human facial skin as a lotion contains about 0.5 to about 10 percent a 6,9-disubstituted purine derivative of this invention (preferably pyranyl kinetin) in a base prepared by mixing 10 parts glycerol monostearate, 10 parts cetyl alcohol, 30 parts spermaceti, 10 parts Tween 20 (polyoxyalkylene derivative of sorbitan monostearate), 10 parts Span 20 (sorbitan monolaurate), 12.5 parts glycerin, and 100 parts water.
(2) **Creams.** The creams are formulated to contain an effective concentration of one or more 6,9-disubstituted purine derivatives. The effective concentration is typically an amount effective to deliver the 6,9-disubstituted purine derivatives to the treated tissue at between about 0.5 to about 10 percent to active cells of the skin, particularly the fibroblasts in the dermis. The creams also contain from about 5 to about 50 percent, preferably from about 10 to about 25 percent, of an emollient and the remainder is water or other suitable non-toxic carrier, such as an isotonic buffer. The emollients, as described above for the lotions, can also be used in the cream compositions. The cream may also contain a suitable emulsifier, as described above. The emulsifier is included is in the composition at a level from about 3 to about 50 percent, preferably from about 5 to about 20 percent.
(3) **Solutions and Suspension.** The solutions are formulated to contain an effective amount of one or more 6,9-disubstituted purine derivatives which is typically an amount effective to deliver the 6,9-disubstituted purine derivatives at between about 0.5 to about 10 percent to active cells of the skin, particularly the fibroblasts of the dermis; the balance is water, a suitable organic solvent or other suitable solvent or buffer. Suitable organic materials useful as the solvent or a part of a solvent system are as follows: propylene glycol, polyethylene glycol (M.W. 200-600), polypropylene glycol (M.W. 425-2025), glycerine, sorbitol esters, 1,2,6-hexanetriol, ethanol, isopropanol, diethyl tartrate, butanediol, and mixtures thereof. Such solvent systems can also contain water.
   These compositions that are formulated as solutions or suspensions may be applied to the skin, or, may be formulated as an aerosol and applied to the skin as a spray. The aerosol compositions further contain from about 25 to about 80 percent, preferably from about 30 to about 50 percent, of a suitable propellant. Examples of such propellants are the chlorinated, fluorinated and chlorofluorinated lower molecular weight hydrocarbons. Nitrous oxide, carbon dioxide, butane, and propane are also used as propellant gases. These propellants are used as understood in the art in a quantity and under a pressure suitable to expel the contents of the container.
(4) **Gels.** Gel compositions can be formulated by simply admixing a suitable thickening agent to the previously described solution or suspension compositions. Examples of suitable thickening agents have been previously described with respect to the lotions.
   The gelled compositions contain an effective amount of one or more 6,9-disubstituted purine derivatives which is typically an amount effective to deliver the 6,9-disubstituted purine derivatives at between about 0.05 to about 10 percent to active cells of the skin, particularly the fibroblasts of the dermis; from about 5 to about 75 percent, preferably from about 10 to about 50 percent, of an organic solvent as previously described; from about 0.5 to about 20 percent, preferably from about 1 to about 10 percent of the thickening agent; the balance being water or other aqueous carrier.
(5) **Solids.** Compositions of solid forms may be formulated as stick-type compositions intended for application to the lips or other parts of the body. Such compositions contain an effective amount of one or more 6,9-disubstituted purine derivatives. The amount is typically an amount effective to deliver the 6,9-disubstituted purine derivatives at between about 0.05 to about 10 percent to active cells of the skin, particularly the fibroblasts of the dermis. The solids also contain from about 50 to about 98 percent, preferably from about 60 to about 90 percent, of the previously described emollients. This composition can further contain from about 1 to about 20 percent, preferably from about 5 to about 15 percent, of a suitable thickening agent, and, if desired or needed, emulsifiers and water or buffers. Thickening agents previously described with respect to lotions are suitably employed in the compositions in solid form.

Other ingredients, such as preservatives, including methyl-paraben or ethyl-paraben, perfumes, dyes or the like, that are known in the art to provide desirable stability, fragrance or color, or other desirable properties, such as shielding from actinic rays from the sun, to compositions for application to the skin may also be employed in any of these types of compositions for topical or other application.

The composition may also include other adverse-age-effect-ameliorative active ingredients, such as retinoids, kinetin, and/or zeatin other than 6,9-disubstituted purine derivatives, but should not include ingredients, such as auxin, that potentiate or induce cell-division inducing properties. Preferred compositions contain, as the only adverse-age-effect-ameliorative ingredient, one or more 6,9-disubstituted purine derivatives.

Compositions for use with human skin preferably may be applied once per day or, if necessary to achieve the desired result, more often, to the areas of the skin for which treatment is sought. It is understood that the precise treatment regimen depends upon the individual treated and may be ascertained empirically depending upon the formulation and, particularly, the age of the treated individual. Any regimen is acceptable as long as the desired age-ameliorating effects are achieved without substantial deleterious or sustained undesirable side effects.

The methods for treating human skin are practiced by applying to the skin, preferably daily, a composition of the invention suitable for human skin treatment, as discussed above, for an indefinite period, generally as long as the person desires to enjoy the amelioration of the adverse effects of aging of the skin. Once daily application to the skin of a composition should be required for at least about a month, and up to at least about a year, depending on the age of the person, the condition of the skin to which the composition is applied, and the concentration of 6,9-disubstituted purine derivative in the composition, before the beneficial effects (e.g., delaying age-related morphological changes in fibroblasts of the basal cell layer of the skin) begin to become apparent at the skin surface. If application of the 6,9-disubstituted purine derivative is terminated, the aging effects ameliorated by the method may again ensue after some time.

For fibroblasts (or other active cells, such as keratinocytes) of the human skin, the method is practiced by applying to the outer surface of the skin a composition that is formulated as a physiologically acceptable cream, ointment, lotion, gel, solution, perfume, solid, or other suitable form for application to the outer surface of the skin, wherein the composition contains one or more 6,9-disubstituted purine derivatives at a concentration effective to deliver to the dermis of the skin an effective concentration effective for ameliorating the adverse effects of aging on active cells (e.g., fibroblasts) in the dermis, whereby the treated skin ages more slowly than untreated skin and/or becomes younger in appearance than prior to treatment as manifested by a reduction in wrinkling and/or sagging or other cosmetic indicators of age. Such concentrations are typically about 0.05 to about 10 percent (preferably about 0.1 to about 2 percent). The precise concentration, which may be empirically determined, is a function of the carrier or delivery vehicle and the form in which the composition is presented to the surface of the skin.

The disclosure also relates to methods for the treatment of the cell senescence and the disease states mentioned above as well as for ameliorating the adverse effects of aging of mammalian cells, especially human cells (and more especially human skin cells). The one or more 6,9-disubstituted purine derivatives of this disclosure can be administered prophylactically or therapeutically in the form of compositions described above and in the effective amounts described above.

As used herein, ameliorating the adverse effect of aging of mammalian cells means that the development of the morphological changes that normally occur with aging in normal mammalian cells in *in vitro* or *in vivo* is slowed, reversed, and/or delayed. The adverse effects of aging also include age related changes in gene expression and protein biosynthesis. The ameliorative effect referred to herein is achieved without substantially increasing the growth rate or total proliferative capacity of the cells that are treated.

Ameliorating the adverse effects of aging on cells, including the effects on *in vitro* and/or *in vivo* cells, may be detected as a delay or reversal of the onset of age-related morphological and phenotypical changes that normally occur with aging of the cells. These changes include the changes detected in tissue culture cells, such as the failure of older cells to respond to exogenous growth factors and/or the high level of autofluorescence found in old cells. As cells age they exhibit an age-dependent loss of proliferative potential. Cultured fibroblast cells that are old display many age-related characteristics, including flattened and irregular morphology, abnormally large size, sparse growth, low cell yield per unit area of culture substratum, a significant frequency of polynucleated cells, difficulty in trypsinization, the inability to grow to confluence, and/or a high rate of production of debris in the culture medium. Young cells exhibit higher responsiveness to growth factors and higher rates of DNA and protein synthesis than old cells. Young mammalian, including human, fibroblast cells in tissue culture appear healthy and clean; possess a regular, long, thin spindle-shaped morphology; are tightly packed in arrays on becoming confluent on culture substrata; do not overgrow one another; seldom have other than one nucleus; and produce little debris in the culture medium. Age related changes *in vivo* include changes in mammalian tissues, such as the development of, or increase in number or depth of, wrinkles, lines, sagging skin, discolorations, blotchiness, leathery, and/or yellowed appearance associated with the cosmetic appearance of the skin as well as the associated changes in the structural and functional integrity of the tissue. The compositions of this invention are effective in improving the overall appearance and condition of the skin, including age-related changes and changes that may not be closely related to aging (e.g., acne, erythema, redness, and the like). For purposes of this invention, such changes that may not be closely related to aging or may even be independent of aging are intended to be included in age-related changes.

The methods of this invention can be used in combination with other therapeutic or cosmetic methods (especially those associated with treatment of human skin). Thus, the present methods can be used in combination with, for example, laser or other light-based treatments or devices.

As used herein, the total proliferative capacity of normal cells, such as fibroblast cells, is a measure of the finite proliferative capacity of cells and refers to the total number of doublings of cell number that a culture of such cells can undergo before growth of the culture ceases and is a function of the age of the donor from which the cells were obtained. Cells obtained from fetal tissue exhibit a greater proliferative capacity in culture than cells obtained from adult tissue.

As used herein, growth rate or rate of proliferation is a measure of the rate at which cells divide. One unit of measure recognized by those of skill in the art is the reciprocal of doubling time. At the end of the life span of a culture of normal cells, the cessation of culture growth appears very quickly, decreasing from near normal values characteristic of young cells to zero in only a few doublings.

As used herein, substantially altering the growth rate or total proliferative capacity means to change beyond the amount that is within normal variation among cells and tissues the rate of cell division or the number of cell doublings. In particular, the growth rate or total proliferative capacity is not altered such that the treated cells are immortalized or undergo malignant transformation. In the case of treated *in vivo* treated cells, the treated tissue does not substantially change size, thickness or develop precancerous or cancerous cells. Methods for assessing the growth rate and total proliferative capacity are known to those of skill in the art. Any such method, including those exemplified herein, may be used to assess the alteration in growth rate or total proliferative capacity.

The 6,9-disubstituted purine derivatives of this invention generally can 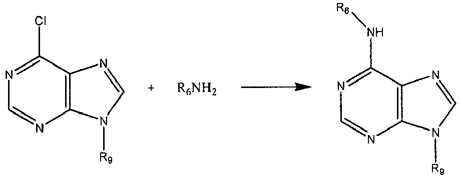 be prepared from the corresponding 6-chloro-9-substituted purines by reaction with the appropriate amine. 6-Chloro-9-(2-tetrahydropyranyl)purine, which can be used to prepare the preferred 6,9-disubstituted purine derivatives of this invention, can be synthesized from 6-chloropurine and 3,4-dihydropyran using p-toluenesulfonic acid according to the literature (Robins et al., J. Am. Chem. Soc. 83, 2574 (1961)). Starting unsubstituted or methoxy-substituted benzylamines, phenylamines and furfurylamines, not commercially available (otherwise obtained via Sigma Aldrich or Fluorochem), can be prepared from the corresponding aldehydes in the presence of suitable metal catalyst. The 6-chloro-9-(2-tetrahydrofuranyl)purine can be synthesized from 6-chloropurine and 3,4-dihydrofuran using p-toluensulfonic acid.

The following examples are included for illustrative purposes. Unless otherwise indicated, all concentrations, ratios, and the like are based on weight.

**Example 1.** This example illustrates the preparation of 6-chloro-9-(2-tetrahydropyranyl)purine. A mixture of 6-chloropurine (60 g, 388 mmol) and tosic acid monohydrate (1 g) in ethylacetate (750 ml) was vigorously stirred at 50°C. 3,4-Dihydropyran (40 ml, 438 mmol) was added dropwise over a 30 min period, maintaining the reaction temperature between 55-60°C (Robins et al., 1961). The solution was stirred for an additional hour during which time it was allowed to cool to room temperature. Concentrated aqueous ammonia (35 ml) was added and the solution stirred for 5 min. Homogenous dark-green solution was subsequently extracted with 2 x 200 ml water. The yellow ethylacetate extract was dried overnight over sodium sulphate and then cool at -20°C. Accrued yellow solid was dried again *in vacuo* over phosphorus pentoxide at 37°C. Yield: 66.9 g (72.2%). MS (ES):

[M+H]⁺ = 239 (100).

**Example 2.** The example also illustrates the preparation of 6-chloro-9-(2-tetrahydrofuranyl)-purine. A mixture of 6-chloropurine (50 g, 323 mmol) and p-toluensulfonic acid (2.5 g, 14.5 mmol) in ethylacetate (200 ml) was vigorously stirred at room temperature. 2,3-Dihydrofuran (37.5 g, 535 mmol) was added dropwise over a 30 min period. The solution was stirred for an additional hour during which 6-chloropurine was completely dissolved (Lewis et al., J. Org. Chem.; 26; 1961; 3837). Subsequently, aqueous ammonia (150 ml) mixed with water in molar ratio 1:1 was added. Homogenous dark-yellow solution was then extracted with 2x100 ml water. Yellow ethylacetate extract was dried overnight over sodium sulphate. Then, the solution was filtrated and evaporated. After vacuum evaporation, a crude yellow oil was dried in vacuo over phospohorus pentoxide at 37°C. Yellow crude product was re-crystallized from petroleum ether. Yield: 80%, yellow solid. Melting point: 92-95°C. TLC (toluen:ethylacetate, 1:2 (v:v), single spot. HPLC purity: >97%. ¹H-NMR (400 MHz, DMSO): 2.05sep(1 H, J = 6.8 Hz); 2.22sep(1 H, J = 6.8 Hz); 2.48m(2H); 3.95q(1 H, J = 7.4 Hz); 4.20qq(1 H, Ja = 7.4 Hz, Jb = 1.9 Hz); 6.40m(1H); 8.78s(1H); 8.80s(1H). MS (ES): [M+H]+ = 225 (100).

**Example 3.** This example illustrates the preparation of 6-furfurylamino-9-(2-tetrahydropyranyl)purine. To furfurylamine (100 g, 91 ml, 1030 mmol,), 30.3 g of 6-chloro-9-(2-tetrahydropyranyl)purine (126.9 mmol) was added. The solid dissolved completely on thorough mixing. The homogeneous solution was heated at 90°C for 60 min and subsequently cooled at room temperature. Colorless, crystalline furfurylamine hydrochloride was immediately deposited and filtered off. The remaining filtrate was evaporated in vacuo. Iso-octane (600 ml) was added to the yellow oil, shaken thoroughly, and allowed to stand at room temperature. The product slowly crystallised over about 1 hour. White solid precipitate was filtered off, washed with ethylether (50 ml), and air dried overnight to remove the solvent. The crude product was recrystalized in methanol. Yield: 24.4 g (81.52 mmol, 64.2%). Melting point: sharp at 144-145°C, no decomposition. TLC (chloroform:methanol (95:5 (v:v))): single spot (Rf=0.35). TLC (chloroform): single spot (Rf=0.34). HPLC purity: 99+%. Elemental analysis % (expected/found): C = 60.19/60.14, H = 5.72/5.70, N = 23.40/23.30.

**Example 4.** The example also illustrates the preparation of 6-furfurylamino-9-(2-tetrahydrofuranyl)purine. To furfurylamine (1456 mg, 15 mmol) placed in 100 ml of n-propanol, 6-chloro-9-(2-tetrahydrofuranyl)purine (2247 mg, 10 mmol) and triethylamine (3.6 ml, 25 mmol) were added. The solid dissolved completely on thorough mixing. The homogeneous solution was heated at 100°C for 3 hrs and subsequently cooled at room temperature. After vacuum evaporation, the resulting material was treated with water (100 ml) and extracted to ethylacetate (100 ml). Ethylacetate extract was evaporated and the residue subsequently washed with 50 ml diethylether. White crude product was re-crystallized in methanol. Yield: 85%, white solid. Melting point: 128-129°C. TLC (CHCl3:methanol, 8:2 (v:v), single spot. HPLC purity: >99%. ¹H-NMR (400 MHz, DMSO): 2.02sep(1 H, J = 6.8 Hz); 2.22sep(1 H, J = 6.8 Hz); 2.42m(2H); 3.91q(1H, J = 7.4 Hz); 4.14q(1H, J = 7.4 Hz); 4.70bs(2H); 6.23dd(1 H, Ja = 3.2 Hz, Jb = 0.9 Hz); 6.26m(1 H); 6.36t(1 H, J = 2.4 Hz); 7.53m(1 H); 8.19bs(1 H); 8.24s(1 H); 8.27s(1 H). MS (ES): [M+H]+= 286 (100).

**Example 5.** This example illustrates the preparation of 6-(4-methoxybenzylamino)-9-(2-tetrahydropyranyl)purine. A mixture of 10 mmol 6-chloro-9-(2-tetrahydropyranyl)purine (prepared from 1546 mg of 6-chloropurine), 12 mmol 4-methoxybenzylamine and 5 ml of triethylamine was refluxed in *n*-propanol for 3 hours. After *n*-propanol vacuum evaporation, the resulting material was treated with water and extracted into ethyl acetate. The ethyl acetate phase was dried over Na₂SO₄, filtered, evaporated and the residue subsequently washed with 30 ml of diethylether. A solid residue was filtered off and the crude product recrystalized from methanol. Yield: 80 %, white solid. Melting Point: 137-138°C. TLC (CHCl₃:methanol (8:2 (v:v))): single spot. HPLC purity: > 98 %. ¹H-NMR (400 MHz, DMSO): 1.55m(2H); 1.68m(1H); 1.93m(2H); 2.26qq (Jₐ = 11.0 Hz, J_{b} = 4.3 Hz); 3.64qq (1 H, Jₐ = 11.0 Hz, J_{b} = 4.3 Hz); 3.70s(3H); 4.00d(1H, J = 11.0 Hz); 4.65s(2H); 5.63dd(1 H, Jₐ= 11.0 Hz, J_{b}= 2.2 Hz); 6.85d(2H, J= 8.8Hz); 7.28d(2H, J= 8.8 Hz); 8.23s (1H); 8.29bs(1H); 8.34s(1H). MS (ES): [M+H]⁺= 340 (100).

**Example 6.** This example illustrates the preparation of 6-(2-methoxybenzylamino)-9-(2-tetrahydropyranyl)purine. A mixture of 6-chloro-9-(2-tetrahydropyranyl)purine (2387 mg, 10 mmol), prepared from 1546 mg of 6-chloropurine, 2-methoxybenzylamine (1470 mg, 12 mmol) and 5 ml triethylamine (35 mmol) was refluxed in n-propanol for 3 hrs. The solid dissolved completely on thorough mixing. After vacuum evaporation of n-propanol, the resulting material was treated with water (100 ml) and extracted to ethylacetate (100 ml). The ethylacetate extract was evaporated and the residue subsequently washed with 50 ml of petrolether. White crude product was re-crystallized in methanol. Yield: 90%, white solid. Melting point: 106-108°C. TLC (CHCl3:methanol, (8:2 (v:v), single spot. HPLC purity: >98%. ¹H-NMR (400 MHz, DMSO): 1.56m(2H); 1.71m(1H); 1.94m(2H); 2.27qq(1H, Ja = 12.3 Hz, Jb = 3.8 Hz); 3.67m(1H); 3.83s(3H); 4.20m(1 H); 4.71 bs(2H); 5.64dd(1 H, Ja = 11.3 Hz, Jb = 1.9 Hz); 6.83tt(1 H, Ja = 7.4 Hz, Jb = 0.9 Hz); 6.97dd(1H, Ja = 8.2 Hz, Jb = 0.7 Hz); 7.14d(1H, J = 7.3 Hz); 7.20tt(1H, Ja = 7.8 Hz, Jb = 1.7 Hz); 8.09s(1H); 8.21 s(1H); 8.36s(1H). MS (ES): [M+H]+= 340 (100).

**Example 7.** This example illustrates the preparation 6-(4-methoxybenzylamino)-9-(2-tetrahydrofuranyl)purine. A mixture of 10 mmol of 6-chloro-9-(2-tetrahydrofuranyl)purine (prepared from 1546 mg of 6-chloropurine), 12 mmol of 4-methoxybenzylamine and 5 ml of triethylamine was refluxed in *n*-propanol for 3 hours. After *n*-propanol vacuum evaporation, the resulting material was consequently treated with water and extracted into ethyl acetate. The ethyl acetate phase was evaporated and the residue subsequently washed with 30 ml of diethylether. The solid residue was filtered off and the crude product crystallized from methanol. Yield: 80 %, white solid. Melting Point: 182-183°C. TLC (CHCl₃:methanol (8:2 (v:v)): single spot. HPLC purity: > 98 %. ¹H NMR (400 MHz, DMSO): 2.02sxt(1H, J = 7.4 Hz); 2.21sxt(1H, J = 7.4 Hz); 2.44m(2H); 3.72s(3H); 3.90q (1 H, J = 7.4 Hz); 4.15q (J = 7.4 Hz); 4.67s(H); 6.28m(1 H); 6.86d(2H, J =8.7 Hz); 7.31d(2H, J = 8.7 Hz); 8.1bs(1H); 8.19s(1H); 8.29s(1H). MS (ES): [M+H]⁺ = 326(100).

**Example 8.** This example illustrates the preparation of 6-(2-methoxybenzylamino)-9-(2-tetrahydrofuranyl)purine. A mixture of 2247 mg (10 mmol) of 6-chloro-9-(2-tetrahydropyranyl)purine (prepared from 1546 mg of 6-chloropurine), 2-methoxybenzylamine (1470 mg, 12 mmol) and 5 ml of triethylamine (35 mmol) was refluxed in n-propanol for 3 hrs. The solid dissolved completely on thorough mixing. After vacuum evaporation of n-propanol, the resulting material was treated with water (100 ml) and extracted to ethylacetate (100 ml). The ethylacetate extract was evaporated and the residue subsequently washed with 50 ml of hexane. White crude product was re-crystallized in methanol. Yield: 90%, white solid. Melting point: 97-99°C. TLC (CHCl3:methanol, 8:2 (v:v), single spot. HPLC purity: >98%. ¹H-NMR (400 MHz, DMSO): 2.02m(1H); 2.21sep(1H, J = 6.8 Hz); 2.43m(2H); 3.83s(3H); 3.91 q(1 H, J = 7.4 Hz); 4.14q(1 H, J = 7.4 Hz); 4.68bs(2H); 6.26m(1 H); 6.83tt(1 H, Ja = 7.4 Hz, Jb = 0.9 Hz); 6.98dd(1 H, Ja = 8.2 Hz, Jb = 0.7 Hz); 7.12d(1H, J = 7.3 Hz); 7.20tt(1H, Ja = 7.8 Hz, Jb = 1.7 Hz); 8.05bs(1H); 8.18s(1H); 8.27s(1H). MS (ES): [M+H]+= 326 (100).

**Example 9.** This example illustrates the preparation of 6-(3-methoxybenzylamino)-9-(2-tetrahydropyranyl)purine. A mixture of 10 mmol of 6-chloro-9-(2-tetrahydropyranyl)-purine, 12 mmol of 3-methoxybenzylamine and 5 ml of triethylamine was refluxed in *n*-propanol for 3 hours. After *n*-propanol vacuum evaporation, the resulting material was treated with water and extracted into ethyl acetate. The ethyl acetate phase was dried over Na₂SO₄, filtered and subsequently evaporated. When the oily residue was treated with 30 ml of *n*-hexane, white powder product formed. The crude product was crystallized from methanol. Yield: 70 %, white solid. Melting Point: 134-135°C. TLC (CHCl₃:CH₃OH (85:15 (v:v)): single spot. HPLC purity: > 99 %. ¹H NMR (400 MHz, DMSO): 1.56m (2H); 1.70m (1 H),1.94m(2H); 2.30qq (1 H, Jₐ = 11.0, J_{b} = 4.3 Hz); 3.67tt (1 H, Jₐ = 11.0 Hz, J_{b} = 4.3 Hz); 3.83s(3H); 4.00d(1 H, J = 11 Hz); 4.71 s(2H); 5.64dd (1 H, Jₐ = 11.0 Hz, J_{b} = 4.3 Hz); 6.83t (1 H, J = 7.7 Hz); 6.97d (1 H, J = 7.7 Hz); 7.14d (J = 7.7 Hz); 7.20tt(1 H, Jₐ = 7.7 Hz, J_{b} = 1.5 Hz); 8.09bs(1 H); 8.21 s(1 H); 8.36s(1 H). MS (ES): [M+H]⁺ = 340 (100).

**Example 10.** This example illustrates the preparation of 6-(4-methoxyphenylamino)-9-(2-tetrahydropyranyl)purine. A mixture of 2387 mg (10 mmol) of 6-chloro-9-(2-tetrahydropyranyl)purine (prepared from 1546 mg of 6-chloropurine), 4-methoxyphenylamine (1803 mg, 15 mmol) and 5 ml of diisopropylamine (35 mmol) was refluxed in n-propanol (100 ml) for 5 hrs. The solid dissolved completely on thorough mixing. After vacuum evaporation of n-propanol, the resulting material was treated with water (50 ml) and extracted to ethylacetate (50 ml). The ethylacetate extract was evaporated and the residue subsequently washed with 50 ml of petrolether. Yield: 90%, white solid. Melting point: 150-151 °C. TLC (ethylacetate:toluene, 3:1 (v:v), single spot. HPLC purity: >98%. ¹H-NMR (400 MHz, DMSO): 1.59m(2H); 1.73m(1H); 1.97m(2H); 2.31qq(1H, Ja = 12.3 Hz, Jb = 3.8 Hz); 3.69m(1 H); 4.02m(1 H); 5.68dd(1 H, Ja = 11.3 Hz, Jb = 1.9 Hz); 6.91 d(1H, J = 9.0 Hz); 7.78d(1H, J = 9.0 Hz); 8.34s(1H); 8.47s(1H); 9.72s(1H). MS (ES): [M+H]+= 326 (100).

**Example 11.** This example illustrates the preparation of 6-(3-methoxybenzylamino)-9-(2-tetrahydrofuranyl)purine. A mixture of 10 mmol of 6-chloro-9-(2-tetrahydrofuranyl)purine (prepared from 1546 mg of 6-chloropurine), 12 mmol of 3-methoxybenzylamine, and 5 ml of triethylamine was refluxed in *n*-propanol for 3 hours. After *n*-propanol vacuum evaporation, the resulting material was treated with water and extracted into ethyl acetate. The ethyl acetate phase was dried over Na₂SO₄, filtered and evaporated. The residue was subsequently washed with 30 ml of *n*-hexane. White powder solid was filtered off and the crude product was crystallized from methanol. After several hours, pure transparent crystals were obtained. Yield: 80 %, white solid. Melting Point: 87-88°C. TLC (CHCl₃:methanol (8:2 (v:v)): single spot. HPLC purity: > 98 %. ¹H NMR (400 MHz, DMSO): 2.02sxt(1 H, J = 7.4 Hz); 2.22sxt(1 H, J = 7.4 Hz); 2.45m(2H); 3.84s(3H); 3.91 q (1 H, J = 7.4 Hz); 4.14q (1 H, J = 7.4 Hz); 4.68s(2H); 6.26m (1 H); 6.83t (1 H, J = 7.7 Hz); 6.98d(1H, J = 7.7 Hz); 7.11d(1H, J =7.7 Hz); 7.20t(1H, J = 7.7 Hz); 8.06bs(1H); 8.17s(1H); 8.27s(1H). MS (ES): [M+H]⁺ = 326 (100).

**Example 12.** This example illustrates the preparation of 6-(2,5-dimethoxybenzylamino)-9-(2-tetrahydropyranyl)purine. A mixture of 2387 mg (10 mmol) of 6-chloro-9-(2-tetrahydropyranyl)purine, 2,5-dimethoxybenzyamine (2006 mg, 12 mmol) and 5 ml of triethylamine (35 mmol) was refluxed in n-propanol for 3 hrs. The solid dissolved completely on thorough mixing. After vacuum evaporation of n-propanol, the resulting material was treated with water (100 ml) and extracted to ethylacetate (100 ml). The ethylacetate extract was evaporated and the residue subsequently washed with 50 ml of diethylether. White solid was filtrated off and dried under vacuum. Yield: 95%, white solid. Melting point: 150-152°C. TLC (ethylacetate:hexane, 1:2 (v:v), single spot. HPLC purity: >98%. ¹H-NMR (400 MHz, DMSO): 1.56m(2H); 1.70m(1 H); 1.94m(2H); 2.27qq (1 H, Ja = 12.3 Hz, Jb = 3.8 Hz); 3.60s(3H); 3.70s(3H); 3.66m(1 H); 3.78s(3H); 4.00m(1H); 4.68bs(2H); 5.64dd(1 H, Ja = 11.2 Hz, Jb = 2.0 Hz); 6.75m(2H); 6.89dd(1 H, Ja = 9.2 Hz, Jb = 1.9 Hz); 8.10bs(1H); 8.21 s(1 H); 8.36s(1 H). MS (ES): [M+H]+= 370 (100).

**Example 13.** The example illustrates the preparation of 6-(2,5-dimethoxybenzylamino)-9-(2-tetrahydrofuranyl)purine. A mixture of 2247 mg (10 mmol) of 6-chloro-9-(2-tetrahydrofuranyl)purine (prepared from 1546 mg of 6-chloropurine), 2,5-dimethoxybenzyamine (2006 mg, 12 mmol) of and 5 ml of triethylamine (35 mmol) was refluxed in n-propanol for 3 hrs. The solid dissolved completely on thorough mixing. After vacuum evaporation of n-propanol, the resulting material was treated with water (100 ml) and extracted to ethylacetate (100 ml). The ethylacetate extract was evaporated and the residue subsequently washed with 50 ml of diethylether. White crude product was re-crystallized in methanol. Yield: 90%, white solid. Melting point: 103-104° C. TLC (ethylacetate:hexane, 1:2 (v:v), single spot. HPLC purity: >98%. ¹H-NMR (400 MHz, DMSO): 2.02m(1 H); 2.21 sep(1 H, J = 6.8 Hz); 2.43m(2H); 3.60s(3H); 3.78s(3H); 3.91 q(1 H, J = 7.4 Hz); 4.14q(1 H, J = 7.4 Hz); 4.65bs(2H); 6.26m(1H); 6.74m(2H); 6.90d(1H, J = 8.8 Hz); 8.10bs(1H); 8.18s(1 H); 8.28s(1 H). MS (ES): [M+H]+= 356 (100).

**Example 14.** The example illustrates the preparation of 6-(2,3,4-trimethoxybenzylamino)-9-(2-tetrahydropyranyl)purine. A mixture of 2387 mg (10 mmol) of 6-chloro-9-(2-tetrahydropyranyl)purine, 2,3,4-trimethoxybenzyamine hydrochloride (2799 mg, 12 mmol) and 8 ml of triethylamine (57 mmol) was refluxed in n-butanol (45 ml) for 3 hrs. The solid dissolved completely on thorough mixing. After vacuum evaporation of n-butanol, the resulting material was treated with water (50 ml) and extracted to ethylacetate (50 ml). The ethylacetate extract was evaporated and the residue subsequently washed with 30 ml of diethylether. White solid was filtrated off and re-crystallized from methanol. Yield: 90%, white solid. Melting point: 142-143°C. TLC (ethylacetate:hexane, 1:2 (v:v), single spot. HPLC purity: >98%. ¹H-NMR (400 MHz, DMSO): 1.57m(2H); 1.72m(1 H); 1.95m(2H); 2.27qq(1 H, Ja = 12.3 Hz, Jb = 3.8 Hz); 3.67m(1 H); 3.73s(3H); 3.75s(3H); 3.84s(3H); 4.00m(1H); 4.66bs(2H); 5.63dd(1 H, Ja = 11.3 Hz, Jb = 1.9 Hz); 6.69d(1 H, J = 8.7 Hz); 6.91d(1H, J = 8.7 Hz); 8.04bs(1H); 8.20s(1H); 8.34s(1H). MS (ES): [M+H]+= 400 (100).

**Example 15.** The example illustrates the preparation of 6-(2,3,4-trimethoxybenzylamino)-9-(2-tetrahydrofuranyl)purine. A mixture of 2247 mg (10 mmol) of 6-chloro-9-(2-tetrahydrofuranyl)purine (prepared from 1546 mg of 6-chloropurine), 2,3,4-trimethoxybenzyamine hydrochloride (2799 mg, 12 mmol) and 8 ml of triethylamine (57 mmol) was refluxed in n-butanol for 3 hrs. The solid dissolved completely on thorough mixing. After vacuum evaporation of n-butanol, the resulting material was treated with water (50 ml) and extracted to ethylacetate (50 ml). The ethylacetate extract was evaporated and the residue subsequently washed with 50 ml of hexane. White crude product was re-crystallized in methanol. Yield: 90%, white solid. Melting point: 140-141 °C. TLC (ethylacetate:hexane, 1:2 (v:v), single spot. HPLC purity: >98%. ¹H-NMR (400 MHz, DMSO): 2.02sep(1 H, J = 6.8 Hz); 2.22sep(1 H, J = 6.8 Hz); 2.44m(2H); 3.73s(3H); 3.75s(3H); 3.84s(3H); 3.91 q(1 H, J = 7.4 Hz); 4.13q(1 H, J = 7.4 Hz); 4.65bs(2H); 6.26m(1 H); 6.70d(1 H, J = 8.6 Hz); 6.90d(1 H, J = 8.6 Hz); 8.03bs(1 H); 8.19s(1 H); 8.26s(1 H). MS (ES):

[M+H]+= 386 (100).

**Example 16.** Several short-term, preliminary studies of the effect of 6-furfurylamino-9-(2-tetrahydropyranyl)purine on cultured human skin fibroblasts cells have been carried out. The initial study involved the addition of 6-furfurylamino-9-(2-tetrahydropyranyl)purine (40 to 400 µM) to human skin fibroblasts cells in a culture. The percentage of attachment of the cells to the surface of the culture flask was not affected after 6 hours of treatment. In the remaining experiments reported in this example, the 6-furfurylamino-9-(2-tetrahydropyranyl)purine was added directly to the culture medium at the same time as the cells were seeded.

A wider range of concentrations (from 0.01 to 500 µM) was tested for determining the effects of 6-furfurylamino-9-(2-tetrahydropyranyl)purine on the survival of cultured human skin fibroblasts after 3 days of treatment. At concentrations between 1 and 200 µM, there may have been a slight stimulation of cell growth and survival. No toxicity or other negative effects were apparent due to treatment of the cells with 6-furfurylamino-9-(2-tetrahydropyranyl)purine.

The effect of 6-furfurylamino-9-(2-tetrahydropyranyl)purine (0 to 400µM) treatment on short term growth of young human fibroblasts was also evaluated. Cell growth was similar in untreated samples and in samples treated with 40, 80, and 200 µM 6-furfurylamino-9-(2-tetrahydropyranyl)purine. At treatment levels of about 400 µM there may have been a slight decrease in growth of cells. Similar experiments were carried out to determine the extent of apoptosis and beta-galactosidase staining in 6-furfurylamino-9-(2-tetrahydropyranyl)purine-treated and untreated cells. There was no suggestion of induction of apoptosis or premature senescence of human fibroblasts treated with the various doses of 6-furfurylamino-9-(2-tetrahydropyranyl)purine.

The effect of 6-furfurylamino-9-(2-tetrahydropyranyl)purine on senescent cells was also examined. An equal number of senescent cells were seeded in separate flasks and then treated with different concentrations of 6-furfurylamino-9-(2-tetrahydropyranyl)purine (0 to 400 µM). Cell numbers were determined after 7 and 14 days of treatment using a Coulter counter after trypsinisation and resuspension of the cells. No negative effects were observed after 7 days. After 14 days of treatment, there may have been a slight negative effect on the survival of senescent cells with 200 µM or more 6-furfurylamino-9-(2-tetrahydropyranyl)purine. At lower concentrations, no negative effects were observed although there might have been a slight increase in the cell number of 6-furfurylamino-9-(2-tetrahydropyranyl)purine-treated senescent cells.

Late passage senescent cells with a life span of more than 95% were also treated with different concentrations of 6-furfurylamino-9-(2-tetrahydropyranyl)purine (0 to 200 µM) to assess age-related changes. The actin staining patterns were examined after three days. Treatment with 6-furfurylamino-9-(2-tetrahydropyranyl)purine generally changed the actin staining pattern from a highly polymerized pattern to a less polymerized filament pattern. Less polymerized and diffused patterns of actin staining are generally associated with youthful characteristics of human fibroblasts. Thus, 6-furfurylamino-9-(2-tetrahydropyranyl)purine appears to revert some of the age-related changes in actin organization in senescent cells.

Senescent human skin fibroblasts were also treated with 6-furfurylamino-9-(2-tetrahydropyranyl)purine (0 to 400µM) and evaluated for reversion of age-related changes. There were significant differences in the appearance of the cells after 7 and 30 days of treatment. Treated cells were generally younger looking in terms of becoming thinner and arranged in arrays after 30 days of treatment. Even at higher concentrations (200 and 400 µM), the 6-furfurylamino-9-(2-tetrahydropyranyl)purine-treated cells appeared to be smaller and have less intracellular debris as compared untreated cells.

Based on these studies, 6-furfurylamino-9-(2-tetrahydropyranyl)purine appears to be well tolerated by young and senescent human skin fibroblasts and to have positive effects in terms of reversion of actin pattern and morphology of senescent cells to relatively younger characteristics. No significant induction of additional growth or cell division was observed.

**Example 17.** *In vitro* cytotoxic activity of several of the 6,9-disubstituted purine derivatives of this invention was determined using a microtiter assay with Calcein AM and a panel of cell lines of different histogenetic and species origin; kinetin was also examined as a control. Because only metabolically active cells cleave Calcein AM (tetraacetoxymethyl ester of calcein), the intracellular concentration of calcein corresponds to the number of vital cells in the culture.

The following cell lines were used: human osteosarcoma (HOS); breast carcinoma MCF-7; human myeloid leukemia K-562; and mouse fibroblasts NIH3T3. The cells were maintained in Nunc/Corning 80cm² plastic tissue culture flasks and cultured in cell culture medium (DMEM with 5 g/l glucose, 2 mM glutamine, 100 U/ml penicillin, 100 µg/ml streptomycin, 10% fetal calf serum, and sodium bicarbonate).

The cell suspensions were prepared and diluted according to the particular cell type and the expected target cell density (2.500-30.000 cells per well based on cell growth characteristics) were added by pipette (80 µl) into 96/well microtiter plates. Inoculates were allowed a pre-incubation period of 24 hours at 37°C and 5% CO₂ for stabilization. Four-fold dilutions of the intended test concentration were added at time zero in 20 µl aliquots to the microtiter plate wells. The test compounds were generally evaluated at six 4-fold dilutions. The highest well concentration used in the present study was 166.7 µM. All samples were examined in triplicates. Incubations of cells with the tested compounds was for 72 hours at 37°C, in 5% CO₂ atmosphere and 100% humidity. At the end of the incubation period, Calcein AM was added to a final concnetration of 1 µg/ml incubated for 1 hour. Fluorescence intensity (FI) was measured with a Labsystem FIA Reader Fluoroscan Ascent (UK). The cell survival (GI₅₀) was calculated using the following equation: GI₅₀ = (FI_{compound exposed well} / mean FI_{control well}) / (mean FI_{control well} - mean FI_{blank}) x 100%. The GI₅₀ value, the drug concentration lethal to 50 % of the cells, was calculated from the obtained dose response curves.

The following results were obtained:

| **Compound** | **GI₅₀ (µmol/l) for Cell Line Tested** | | | |
|---|---|---|---|---|
| | **HOS** | **K-562** | **MCF7** | **NIH-3T3** |
| Kinetin | >166.7 | 164.1 | >166.7 | 155.1 |
| 6-furfurylamino-9-(2-tetrahydropyranyl)purine | >166.7 | >166.7 | >166.7 | >166.7 |
| 6-(3,5-dimethoxybenzylamino)-9-(2-tetrahydropyranyl)purine | >166.7 | - | >166.7 | >166.7 |
| 6-(4-methoxybenzylamino)-9-(2-tetrahydropyranyl)purine | >166.7 | >166.7 | >166.7 | > 166.7 |
| 6-(2,3-dimethoxybenzylamino)-9-(2-tetrahydropyranyl)purine | >166.7 | - | >166.7 | >166.7 |
| 6-(3-methoxybenzylamino)-9-(2-tetrahydrofuranyl)purine | >166.7 | - | >166.7 | >166.7 |
| 6-(4-methoxybenzylamino)-9-(2-tetrahydrofuranyl)purine | >166.7 | - | >166.7 | > 166.7 |

The 6,9-disubstituted purine derivatives of this invention showed minimal or no toxicity to cells in concentrations up to 166.7 µM and thus are suitable for cosmetic applications.

**Example 18.** *In vitro* cytotoxic activity of several of the 6,9-disubstituted purine derivatives of this invention against human diploid fibroblasts was determined by standard MTT assay optimized for 96-well plates. The assay is based on spectrofotometric measurement of the product of metabolic conversion of MTT by mitochondria of living cells.

Human forskin fibroblasts (cell line BJ) at middle passage were maintained in 75 cm² plastic tissue culture flasks and cultured in cell culture medium (DMEM with 5 g/l glucose, 2 mM glutamine, 100 U/ml penicillin, 100 µg/ml streptomycin, 10% fetal calf serum, and sodium bicarbonate).

About 5,000 cells were seeded per well of the 96-well plate. After 24 hours the cell culture medium was replaced with the cell culture medium containing a test compound. The test compounds were evaluated at six 2-fold dilutions. The highest concentration used in the present study was usually 200 µM. In the case of compounds with limited solubility in the culture medium the highest concentration was adjusted. Every test compound was examined in five replicates. Cells with the test compounds were incubated for 72 hours at 37°C in 5% CO₂ atmosphere and 100% humidity. At the end of the incubation period, the culture medium was replaced with the cell culture medium containing MTT (0.5 mg/ml) and cells were incubated for another 3 hours. The formazan formed was solubilized by DMSO and absorbance at 570 nm was measured. Ability of the tested compounds to inhibit MTT reducing activity of the cells was calculated as IC = (A_{compound exposed well} - mean A_{blank})/ (mean A_{control well} - meanA blank) x 100%. IC₁₀, the drug concentration causing 10% decrease in MTT reducing activity, was calculated from the obtained dose response curves.

The following results were obtained:

| **Compound** | **Maximum Concentration Tested (µmol/l)** | **IC₁₀ (µmol/l)** |
|---|---|---|
| 6-furfurylamino-9-(2-tetrahydropyranyl)purine | 200 | >200 |
| 6-furfurylamino-9-(2-tetrahydrofuranyl)purine | 200 | >200 |
| 6-(4-methoxybenzylamino)-9-(2-tetrahydropyranyl)purine | 100 | >100 |
| 6-(4-methoxybenzylamino)-9-(2-tetrahydrofuranyl)purine | 100 | >100 |
| 6-(3-methoxybenzylamino)-9-(2-tetrahydrofuranyl)purine | 100 | >100 |

| **Compound** | **Maximum Concentration Tested (µmol/l)** | **IC₁₀ (µmol/l)** |
|---|---|---|
| 6-(2,4-dimethoxybenzylamino)-9-(2-tetrahydropyranyl)purine | 25 | >25 |
| 6-(3,5-dimethoxybenzylamino)-9-(2-tetrahydrofuranyl)purine | 200 | >200 |
| 6-(3,4-dimethoxybenzylamino)-9-(2-tetrahydropyranyl)purine | 200 | >200 |
| 6-(3,4-dimethoxybenzylamino)-9-(2-tetrahydrofuranyl)purine | 200 | >200 |
| 6-(2,3,4-trimethoxybenzylamino)-9-(2-tetrahydropyranyl)purine | 37.5 | >37.5 |
| 6-(2,3,4-trimethoxybenzylamino)-9-(2-tetrahydropyranyl)purine | 200 | >200 |
| 6-(2,4,5-trimethoxybenzylamino)-9-(2-tetrahydropyranyl)purine | 200 | >200 |
| 6-(2,4,5-trimethoxybenzylamino)-9-(2-tetrahydropyranyl)purine | 200 | >200 |
| 6-(2,4,6-trimethoxybenzylamino)-9-(2-tetrahydropyranyl)purine | 50 | >50 |
| 6-(3,4,5-trimethoxybenzylamino)-9-(2-tetrahydropyranyl)purine | 100 | >100 |
| 6-(3,4,5-trimethoxybenzylamino)-9-(2-tetrahydropyranyl)purine | 200 | >200 |

The tested 6,9-disubstituted purine derivatives of this invention showed no detrimental effect on mitochondrial activity and cell viability in a wide range of concentrations and thus are suitable for cosmetic applications.

**Example 19.** Inhibition of senescence by several of the 6,9-disubstituted purine derivatives of this invention was determined; kinetin was also evaluated as a control. Human diploid fibroblasts (HCA cells of various passage levels: passage 25 - designated HCA25; passage 45 - designated HCA45; and passage 80 - designated HCA80) were stained for β-galactosidase activity. The medium used for the cell cultivation was removed, the cells were washed twice in PBS, and fixed in 2-3 ml of fixing solution comprised of a 2 % formaldehyde and 0.2 % glutaraldehyde in PBS. The cells were incubated at room temperature for 5 minutes, and then washed twice with PBS. The cells were subsequently incubated at 37°C (without CO₂) for 1 to 16 hours in 2-3 ml of the solution comprising potassium ferricyanide (5 mM), potassium ferrocyanide (5 mM), MgCl₂ (2 mM), X-gal (5-bromo-4-chloro-3-indolyl-β-D-galactopyranoside) (1 mg/ml), in citric/phosphate buffer (pH 6.0). The test compounds (about 50 µM) were added to the medium at each passage. Following this incubation period, the cell samples were observed in order to detect the presence of blue cells, indicating that X-gal had been cleaved (positively senescent cells). In this experiment, only senescent cells were stained blue due to the action of β-galactosidase on the substrate.

The results are presented below:

| **Compound** | **Senescent Cells (%)** | | |
|---|---|---|---|
| | **HCA25** | **HCA50** | **HCA80** |
| Kinetin | 3 | 5 | 38 |
| 6-(2-methoxybenzylamino)-9-(2-tetrahydropyranyl)purine | 4 | 6 | 22 |
| 6-(3-methoxybenzylamino)-9-(2-tetrahydropyranyl)purine | 5 | 5 | 24 |
| 6-(4-methoxybenzylamino)-9-(2-tetrahydropyranyl)purine | 4 | 3 | 26 |
| 6-(2,4-dimethoxybenzylamino)-9-(2-tetrahydropyranyl)purine | 4 | 6 | 25 |
| 6-(2,3,4-trimethoxybenzylam ino)-9-(2-tetrahydropyranyl)purine | 4 | 5 | 31 |
| 6-(furfurylamino)-9-(2-tetrahydrofuranyl)purine | 3 | 4 | 12 |
| 6-(2,4-dimethoxyphenylamino)-9-(2-tetrahydropyranyl)purine | 4 | 4 | 29 |
| 6-(3,4-dimethoxyphenylamino)-9-(2-tetrahydropyranyl)purine | 5 | 7 | 28 |
| 6-(2,4,5-trimethoxyphenylamino)-9-(2-tetrahydropyranyl)purine | 5 | 4 | 25 |

The 6,9-disubstituted purine derivatives were generally more effective than kinetin in retaining lower levels of senescent cells after 80 passages.

Reference Example **20.** Anti-inflammatory activity of several of the 6,9-disubstituted purine derivatives of this invention was determined; kinetin was also evaluated as a control. Rat C6 glioma (ATCC No. CCL107) was cultivated in monolayer in serum-free chemically defined medium containing Ham's F10/minimal essential medium (1:1 v/v), 2 mM L-glutamine, 1 % (v/v) minimal essential medium vitamins (100x), 1 % (v/v) minimal essential medium nonessential amino acids (100x), 100 U/ml penicillin, 100 mg/ml streptomycin, and 30 nM sodium selenite. Incubation was performed at 37°C in a humidified atmosphere. The assays were performed in the logaritmic growth phase at a density of 2.5x10⁵ cells/cm². Intracellular cAMP synthesis was induced by addition of 5 mM (-)-isoproterenol; various amounts of test compounds were added at the same time as the (-)-isoproterenol. After 30 min incubation at 37°C, the medium was removed and the cellular amount of cAMP was determined using the cAMP-enzyme immunoassay kit from Amersham. The I₅₀ value was determined from a dose-response curve in duplicate.

The following results were obtained:

| **Compound** | **Anti-inflammatory Activity** | |
|---|---|---|
| | **I₅₀ (µM)** | **Effect** |
| Kinetin | - | Not active |
| 6-furfurylamino-9-(2-tetrahydropyranyl)purine | 13 | Inhibition |
| 6-phenylamino-9-(2-tetrahydropyranyl)purine | 45 | Inhibition |
| 6-(3-methoxybenzylamino)-9-(2-tetrahydropyranyl)purine | 7 | Inhibition |
| 6-(3,5-dimethoxybenzylamino)-9-(2-tetrahydropyranyl)purine | 11 | Inhibition |

The 6,9-disubstituted purine derivatives demonstrated anti-inflammatory activity. Kinetin was inactive in the test protocol.

**Example 21.** A number of tests related to the safety of 6-furfurylamino-9-(2-tetrahydropyranyl)purine have been carried out using conventionally accepted protocols and procedures. The results of these studies are summarized herein.

**Ames Test.** Tests were carried out using DMSO as solvent and dose levels of 6-furfurylamino-9-(2-tetrahydropyranyl)purine at 2.5, 5.0, 15, 50, 500, 1500, and 5000 µg/plate based upon standard protocol and procedures (Ames et al., Mutation Research, 31, 347-364 (1975); Maron et al., Mutation Research, 113, 173-215 (1983)). Using *Salmonella typhimurium* histidien auxotrophs TA98 and TA100 in the presence and absence of Aroclor-induced rat liver S9, no positive mutagenic responses were observed with 6-furfurylamino-9-(2-tetrahydropyranyl)purine at the levels tested.

***In Vitro* Chromosome Aberration Screening Assay.** 6-Furfurylamino-9-(2-tetrahydropyranyl)purine was tested in the chromosome aberration screening assay using Chinese hamster ovary (CHO) cells in both the absence and presence of an Acroclor-induced S9 activation system to evaluate its clastogenic potential. DMSO was used as the solvent and the dose rate ranged from 0.272 to 272 µg/ml. The percentage of cells with structural or numerical aberrations in the 6-furfurylamino-9-(2-tetrahydropyranyl)purine treated samples was not significantly increased above that of solvent control samples. Based on this study, 6-furfurylamino-9-(2-tetrahydropyranyl)purine was concluded to be negative for the induction of structural and numerical chromosome aberrations in CHO cells.

**Chorioallantoic Membrane Vascular Assay.** The potential of 6-furfurylamino-9-(2-tetrahydropyranyl)purine for ocular irritation was assessed using the procedure described in Bagley et al., Alternative Methods in Toxicology, Vol. 6 in Progress in In Vitro Toxicology, 131-138 (1988). The chorioallantoic membrane of White Leghorn eggs, incubated for 14 days, were dosed with 40p1 of the test compound (and three lower concentrations in distilled water) and then further incubated for about 30 minutes at which time the chorioallantoic membrane was examined for vascular hemorrhage, capillary injection, and/or the presence of ghost vessels. The RC₅₀ value (calculated concentration producing a positive reaction in 50% of treated eggs) was 105%. Under the conditions of this assay, a RC₅₀ value of less than 1 % would be considered an irritant whereas a RC₅₀ value of greater than 3% would be considered a non-irritant. Thus, based on this assay, the test compound was found to be a non-irritant.

**Epiderm MTT Viablity Assay.** Using MatTek Corporation's EpiDerm™ System (consisting of normal, human-derived epidermal keratinocytes (NHEK) which have been cultured to form a multilayered, highly differentiated model of the human epidermis), it was determined that 6-furfurylamino-9-(2-tetrahydropyranyl)purine would be expected to be classified as non-irritating.

**Acute Oral Toxicity.** Female Wistar rats were orally dosed with 2000 mg/kg of 6-furfurylamino-9-(2-tetrahydropyranyl)purine and then observed ½, 1, 2, 3, and 4 hours after dosing and thereafter once a day for 14 days of toxicity and pharmacological effects; all animals were humanely sacrificed using CO₂ after the study and examined for gross pathology. All animals survived the oral dose; weight changes during the study were normal; and necropsy results were normal.

**Human Repeated Insult Patch Test.** Test gel (about 0.2 ml) containing about 0.1 percent 6-furfurylamino-9-(2-tetrahydropyranyl)purine was placed onto a 2 cm square occlusion patch was placed on each subjects back and remained for 24 hours. This procedure was repeated every Monday, Wednesday, and Friday until nine (9) applications had been made on the same area (induction phase). The area was examined before each new application. About 10-14 days after the ninth patch had been removed, a challenge patch (challenge phase) on a new area of the back and then examined 24 and 72 hours thereafter for reactivity. Skin responses were based on a six-point scale (0 = no evidence of effect; + = barely perceptible; 1 = mild; 2 = moderate; 3 = marked; and 4 = severe).

Fifty-two (52) subjects completed the induction and challenge phases (total of 10 applications per subject). Of subjects who completed the study, only one subject had a barely perceptible rating (+) after application in the challenge phase (at 24 hours only; when examined at 72 hours there was no evidence of effect); this one observed response was not considered evidence of irritation or allergic in nature. All other subjects did not present any evidence of irritation (i.e., 0 on the scale) during the induction or challenge phases. No evidence of induced allergic contact dermatitis or other irritation in human subjects was observed.

**Example 22.** The safety and efficacy of the 6,9-disubstituted purine derivatives of this invention as topical skin anti-aging treatments were examined using the hairless mouse model (a well established model for studying the treatments of photoaging). Female SKH-1 hairless mice (5 weeks old, 20-25 grams, Charles River Laboratories, Wilmington, MA) were individually housed in filter-top cages, and acclimated for 5-7 days after delivery. The mice were divided into treatment groups (n=6), including two different control groups (untreated control, vehicle control) and a therapeutic control (commercially available 0.05% trans-retinoic acid cream). Water and mouse chow are provided *ad libitum.*

The experimental groups (6 mice per group) included the following treatments: (1) Untreated control; (2) Vehicle control (MillCreek Lotion); (3) Kinetin (0.1 % in MillCreek Lotion); (4) 6-Furfurylamino-9-(2-tetrahydropyranyl)purine (0.1 % in MillCreek Lotion); and (5) Therapeutic control (0.05% trans-retinoic acid cream). The various treatments were applied daily (Monday-Friday) for 3 weeks to the dorsal skin (about 2 cm x 2 cm) of the hairless mice at a dosage rate of about 20 mg.

At baseline (prior to the 1 st treatment), and weekly, the dorsal skin was measured for transepidermal water loss (TEWL), skin moisture content, and skin elasticity. The possible effect of these topical formulations on epidermal cell proliferation was investigated using bromodeoxyuridine as an immunohistochemical marker of cell proliferation. Histological examination of the treated and control skin was also be used to determine cutaneous effects of these topical formulations.

Daily examinations were performed to assess the possible occurrence of erythema (irritation) of the sites using established scoring criteria, wherein Grade 0 = no response; Grade 1 = very slight redness; Grade 2 = slight redness; Grade 3 = moderate redness/irritation; Grade 4 = severe redness/irritation; Grade 5 = very severe redness/irritation; and Grade 6 = necrosis.

The measurements of skin moisture content and skin elasticity are important noninvasive methods used to characterize the effects of moisturizers and anti-wrinkle effects on skin. A DermaLab™ combination instrument was used to measure the skin moisture content and elasticity of the target skin sites at baseline and at weekly intervals. This instrument was equipped with dual probes which are placed on the skin surface and a quantitative measurement taken of the respective parameters and the measurements recorded on an integrated computer.

All animal groups were injected with bromodeoxyuridine (100 mg/kg) I.P. 4 hours after the final application. Animals were then sacrificed by CO₂ inhalation 3 hours later followed by cervical dislocation. The test sites were excised and 6 mm punch biopsies were obtained from each treatment site and from untreated control sites. Biopsies were placed into labeled vials containing 4% neutral buffered formalin for paraffin embedding and anti-BrdU staining. Paraffin sections were cut to a 5 µM thickness and stained using the BrdU immunohistochemistry kit (X1545K from Exalpha Biologicals, Inc.) and a standard staining protocol. The slides were weakly counter stained in Mayer's hematoxylin and scored under a light microscope for the number of BrdU-positive cells per mm of epidermis for each section.

Skin biopsies were taken of each treatment site and untreated control skin. Biopsies were fixed in 4% neutral buffered formalin, embedded in paraffin, and stained with hematoxylin and eosin. The stained skin sections were examined to determine the effects of the treatment on epidermal, dermal, and stratum corneum histology. Biopsies were also microscopically examined for inflammatory cells.

**Skin Irritation.** The test products were well tolerated with extended treatment for 3 weeks. Only the trans-retinoic acid cream caused significant irritation (Grade 3) following 1 to 3 weeks of treatment. All other treatments were below Grade 1.5 with 6-furfurylamino-9-(2-tetrahydropyranyl)purine treatment falling below Grade 1 over the 3 week period.

**Skin Moisture Content.** The therapeutic control showed a significant decrease in skin conductance and, thus, a decreased moisture content of the skin. In contrast, the test compounds and the vehicle alone produced a gradual increase in skin moisture content. At week 3 the mean moisture content of the all test compounds was greater than the vehicle or untreated control. Generally, 6-furfurylamino-9-(2-tetrahydropyranyl)purine treatment produced one of the highest increases in skin moisture of the compounds examined.

**Skin Elasticity.** No significant changes in the skin elasticity of the treatment groups were observed during the 3 week test period for any of the test groups. Thus, the skin elasticity of the treatment groups was comparable to that of the untreated control and vehicle only treatment groups.

**Bromodeoxyuridine (BrdU) Staining.** Bromodeoxyuridine staining of the epidermis was measured to determine the effect of the test compounds on epidermal cell proliferation. There was no statistical difference in epidermal BrdU staining in the test compounds as compared to the untreated or vehicle control or any differences in BrdU staining with the test compounds. The therapeutic control-treated tissues had no epidermal BrdU staining, but some localized areas of staining in the dermis, possibly related to retinoids-induced inflammation.

Tissue biopsies were obtained at the completion of the study after 3 weeks of treatment. The histological evaluation showed normal "healthy" appearing skin with all test compounds. In contrast, the therapeutic control showed marked increased thickness of the epidermis and inflammatory changes in the dermis. The skin compartment thickness of the H&E stained biopsies was measured by optical microscopy. The thickness of the epidermis, dermis and stratum corneum measured after 3 weeks of treatment was comparable to that of the vehicle and untreated control. In contrast, the therapeutic control increased both epidermal and dermal thickness.

These results provide evidence for both the safety and efficacy of 6-furfurylamino-9-(2-tetrahydropyranyl)purine for use to improve the cosmetic appearance and to preserve the vitality of aging skin without irritation.

**Example 23.** A clinical study has been conducted to determine the cosmetic efficacy and subjects' tolerance of topical 6-furfurylamino-9-(2-tetrahydropyranyl)purine (0.10%) applied twice daily for 12 weeks to improve the clinical signs and symptoms of photodamaged facial skin. Forty female volunteer subjects age 40 to 65 with mild to moderate signs of photo damaged facial skin were enroled in the study. Thirty four subjects competed the study; the mean age of subjects competing the study was about 54 years. Subjects were instructed to apply the test product to the entire facial skin twice daily (i.e., early morning and approximately 1 hour before bedtime) for 12 consecutive weeks. Subjects were also instructed that, other than sunscreens or mild cleansers and use of color cosmetics, no other topical skin care products or medications were to be used on the face during the study. The test product comprised 0.1 percent 6-furfurylamino-9-(2-tetrahydropyranyl)purine in MillCreek Lotion as vehicle.

Subjects were assessed at weeks 2, 4, 8, and 12. The treated facial skin was evaluated for clinical signs of skin aging (e.g., coarse and fine wrinkles, roughness, mottled hyperpigmentation) at study entry (baseline) as well as 2, 4, 8 and 12 weeks. The subjects' self-assessment of improvement over baseline (wrinkles, texture, blotchiness, color, and overall improvement) were also obtained. In addition, transepidermal water loss (TEWL) and skin moisture measurements were taken on the cheeks of all subjects.

**Transepidermal Water Loss (TEWL).** TEWL refers to the amount of water vapor loss through the stratum corneum. An increase in TEWL values suggests either evaporative water loss (e.g., sweating or evaporation) or skin barrier damage. A decrease in TEWL values suggests either improvement in barrier function or the presence of a barrier over the skin. TEWL measurements were taken using a TEWL meter (Courage & Khazaka, Köln, Germany) having composed of a probe (containing humidity and temperature sensors) connected to a central processing unit. For each subject, the probe was placed in the center of both cheeks and measurements taken in duplicate.

**Skin Moisture Measurements.** An indirect measurement of the skin's moisture content was made by measuring changes in the skin's electrical properties. Impedance based capacitance measurements were taken on the skin using a NOVA DPM 9003^{®} (NOVA Technologies, Gloucester, MA, USA). The instrument performs measurements at varying preselected frequencies (up to 1 MHz) of the applied alternating current. A value directly related to capacitance is determined in arbitrary units; a higher value indicates a greater capacitance which indicates an increased level of moisture at the test site. Measurements in triplicate were taken in the center of both cheeks for each test subject.

**Expert Assessment of Subject's Facial Skin.** At the baseline (Day 0) visit, an investigator evaluated each subject's face for the presence of fine wrinkles, coarse wrinkles, roughness, mottled hyperpigmentation, and other parameters using a five-point scale (0 = None; 1 = Minimal; 2 = Mild; 3 = Moderate; and 4 = Severe). The overall severity of skin photodamage (wrinkles, roughness and mottled hyperpigmentation) was evaluated using a 10 point scale (0 = None; 1 - 3 = Mild; 4 - 6 = Moderate; and 7 - 9 = Severe); subjects with baseline severity scores greater than 6 were excluded from the study.

On each subsequent visit, the investigator evaluated overall improvement compared to baseline using a 6-point scale (1 = excellent improvement; 2 = marked improvement; 3 = moderate improvement; 4 = slight improvement; 5 = no improvement; and 6 = worse).

**Subject's Perception of Efficacy.** At each subsequent visit, the subjects were asked to complete a self-assessment questionnaire to assess the improvement from baseline for skin texture, skin color, blotchiness (i.e., brown spots), fine wrinkles; and overall improvement using a five-point scale (1 = much improved; 2 = somewhat improved; 3 = no change; 4 = somewhat worse; and 5 = much worse).

**Results.** The following test results, averaged over all subjects and reported as mean values, were obtained.

| | **Baseline** | **Week 2** | **Week 4** | **Week 8** | **Week 12** |
|---|---|---|---|---|---|
| TEWL (g/m²/hr) | 13.15 | 12.89 | 11.70* | 13.08 | 9.54* |
| Change Relative to Baseline | | -1.98% | -9.62% | 0.11% | -27.79% |
| Skin Moisture (arbitrary units) | 118.03 | 125.37* | 141.30* | 158.04* | 165.42* |
| Change Relative to Baseline | | 6.22% | 20.96% | 35.10% | 41.21% |

| | | | | | |
|---|---|---|---|---|---|
| * Significant difference (p≤0.05) as compared to baseline. | | | | | |

A decrease in TEWL values indicates an improvement in skin barrier function; an increase in TEWL values may indicate a disruption in the barrier properties. An increase in skin moisture indirectly indicates an increase in moisture levels.

For the investigator's assessment of general skin conditions, the following results were obtained using a five-point scale (0 = None; 1 = Minimal; 2 = Mild; 3 = Moderate; and 4 = Severe).

| | **Baseline** | **Week 2** | **Week 4** | **Week 8** | **Week 12** |
|---|---|---|---|---|---|
| Fine Wrinkles | 2.23 | 2.18 | 1.97* | 1.74* | 1.62* |
| Change Relative to Baseline | | -1.28% | -11.25% | -21.79% | -27.63% |
| Coarse Wrinkles | 2.25 | 2.35 | 2.22 | 2.20 | 2.03* |
| Change Relative to Baseline | | 4.44% | -4.76% | -7.23% | -13.75% |
| Roughness | 1.68 | 1.05* | 0.58* | 0.23* | 0.26* |
| Change Relative to Baseline | | -37.31% | -63.79% | -85.71% | -83.64% |
| Mottled Hyperpigmentation | 1.73 | 1.70 | 1.50* | 1.23* | 1.03* |
| Change Relative to Baseline | | -1.45% | -15.63% | -30.65% | -40.68% |
| Skin Irritation | 0 | 0 | 0 | 0 | 0 |
| Change Relative to Baseline | | 0 | 0 | 0 | 0 |
| Acne | 0.63 | 0.60 | 0.53 | 0.53 | 0.32* |
| Change Relative to Baseline | | -4.00% | -13.64% | -9.52% | -45.00% |
| Erythema^{†} | 0.60 | 0.40^{††} | 0.31* | 0.20* | 0.21* |
| Change Relative to Baseline | | -33.33% | -47.62% | -66.67% | -66.67%% |

| | | | | | |
|---|---|---|---|---|---|
| * Significant difference (p≤0.05) as compared to baseline. ^{†} If data was restricted to only subjects having signs of erythema at baseline, there was a significant difference at all time periods. When data was restricted to only subjects having no signs of erythema at baseline, there was no significant increase at any time period, indicating no inducement of visible signs of erythema. ^{††} Highly suggestive (p=0.056) of significant decrease in erythema at week 2. | | | | | |

A negative percentage change in the parameters in the above table represents an improvement in the relevant parameter. A significant improvement was noted by the expert evaluators in one or more time periods for fine and coarse wrinkles, roughness, mottled hyperpigmentation, acne, and erythema. No skin irritation was observed.

The investigator's assessment of overall skin condition based on cosmetic appearance related to skin aging using a 10 point scale (0 = None; 1 - 3 = Mild; 4 - 6 = Moderate; and 7 - 9 = Severe) was as follows: 4.10 at baseline; 4.05 at 2 weeks (-1.22% change relative to baseline); 3.56 at 4 weeks (-15.23% change relative to baseline); 3.20 at 8 weeks (-24.32% change relative to baseline); and 3.03 at 12 weeks (-27.97% change relative to baseline). The differences noted at weeks 4, 8, and 12 were significant (p≤0.001). A negative percentage change in overall skin condition represents an improvement in cosmetic appearance related to skin aging.

The investigator's assessment of overall improvement in skin condition (relative to baseline) using a 6-point scale (1 = excellent improvement; 2 = marked improvement; 3 = moderate improvement; 4 = slight improvement; 5 = no improvement; and 6 = worse) was as follows: 4.95 at 2 weeks; 4.44 at 4 weeks (-10.61% change relative to 2 week data); 4.11 at 8 weeks (-16.67% change relative to 2 week data); and 4.12 at 12 weeks (-17.16% change relative to 2 week data). The differences noted at weeks 4, 8, and 12 were significant (p≤0.001). A negative percentage change in overall improvement represents an improvement in skin conditions.

The results of the subjects' self assessment of skin conditions during the study using a five-point scale (1 = much improved; 2 = somewhat improved; 3 = no change; 4 = somewhat worse; and 5 = much worse) were as follows:

| | **Week 2** | **Week 4** | **Week 8** | **Week 12** |
|---|---|---|---|---|
| Skin Texture | 2.21 | 2.03* | 1.91* | 1.74* |
| Change Relative to 2 Week Data | | -8.97% | -15.58% | -23.38% |
| Skin Color | 2.74 | 2.50* | 2.23* | 2.12* |
| Change Relative to 2 Week Data | | -5.32% | -18.28% | -22.58% |
| Blotchiness/Brown Spots | 2.70 | 2.58 | 2.26* | 2.15* |
| Change Relatl've to 2 Week Data | | -6.19% | -18.95% | -23.16% |
| Fine Wrinkles | 2.62 | 2.11* | 2.00* | 1.88* |
| Change Relative to 2 Week Data | | -18.68% | -23.60% | -28.09% |
| Overall Improvement | 2.36 | 2.14 | 1.97* | 1.91* |
| Change Relative to 2 Week Data | | -6.17% | -16.25% | -18.75% |

| | | | | |
|---|---|---|---|---|
| * Significant difference (p≤0.05) as compared to two week data. | | | | |

A negative percentage change in the parameters in the above table represents an improvement in the relevant parameter. Subjects reported a significant improvement in one or more time periods for skin texture, skin color, blotchiness, brown spots, and fine wrinkles. Additionally, subjects reported a significant improvement at weeks 8 and 12 in overall condition and appearance of their skin.

Overall, subjects experienced a significant improvement in overall skin condition and a significant reduction in the adverse effects associated with aging over the study period based on both expert evaluation and self assessment. Skin irritation or other adverse effects were not observed.

**Example 24.** This example compares the clinical data for topically applied 6-furfurylamino-9-(2-tetrahydropyranyl)purine (0.10%) taken from Example 23 with similar clinical data generated for topically applied kinetin (0.1 %) from an earlier study. The protocol for the earlier clinical trial for kinetin was similar to the protocol as described in Example 23 for the inventive compound; thirty two female volunteers completed the earlier kinetin study. This comparison is based on the expert evaluations and self evaluations of skin condition for eight and twelve week data for some of the parameters evaluated in both studies.

The comparisons based on expert evaluation are as follows:

| | **Mean Improvement from Baseline** | | | |
|---|---|---|---|---|
| | **Eight Week Data** | | **Twelve Week Data** | |
| | **Kinetin** | **Inventive Compound** | **Kinetin** | **Inventive Compound** |
| TEWL | 13% | 1% | 15% | 28% |
| Fine Wrinkles | 2% | 22%* | 6%* | 28%* |
| Coarse Wrinkles | 4% | 7% | 4% | 14%* |
| Roughness | 35% | 86%* | 52%* | 84%* |
| Mottled Hyperpigmentation | 25%* | 31%* | 25%* | 41%* |
| Overall Skin Condition | 3% | 24%* | 4%* | 28%* |

| | | | | |
|---|---|---|---|---|
| * Clinically significant improvements (p≤0.05) | | | | |

The comparisons based on the subject's self assessment are as follows:

| | **Subjects Reporting Improvement from Baseline** | | | |
|---|---|---|---|---|
| | **Eight Week Data** | | **Twelve Week Data** | |
| | **Kinetin** | **Inventive Compound** | **Kinetin** | **Inventive Compound** |
| Texture | 77% | 83% | 87% | 88% |
| Fine Wrinkles | 57% | 83% | 71% | 88% |
| Blotchiness | 50% | 60% | 74% | 63% |
| Color | 53% | 66% | 71% | 68% |

## Claims

1. A method for improving the cosmetic appearance of mammalian epidermal cells, comprising the use of a compound of formula (I) or a pharmaceutically acceptable salt thereof: 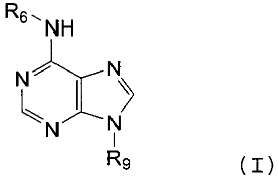 wherein R₆ is furfuryl, methoxy-substituted furfuryl, phenyl, methoxy-substituted phenyl or methoxy-substituted benzyl, and R₉ is 2-tetrahydropyranyl or 2-tetrahydrofuranyl.

2. The method according to claim 1, wherein the mammalian cells are human skin cells.

3. The method according to claim 1 or 2, wherein in formula (I) R₆ is furfuryl or methoxy-substituted furfuryl and R₉ is 2-tetrahydropyranyl.

4. The method according to claim 3, wherein R₆ is furfuryl.

5. A method for improving the cosmetic appearance of human skin cells, comprising the use of a composition comprising a compound of formula (I) or a pharmaceutically acceptable salt thereof: 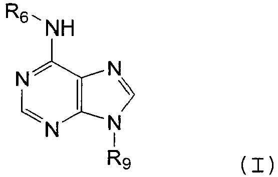 wherein R₆ is furfuryl, methoxy-substituted furfuryl, phenyl, methoxy-substituted phenyl or methoxy-substituted benzyl, and R₉ is 2-tetrahydropyranyl or 2-tetrahydrofuranyl.

6. The method according to claim 5, wherein the human skin cells are in a living human being.

7. The method according to claim 5 or 6, wherein in formula (I) R₆ is furfuryl or methoxy-substituted furfuryl and R₉ is 2-tetrahydropyranyl.

8. The method according to claim 7, wherein R₆ is furfuryl.

9. The method according to any of claims 5-8, wherein the content of the compound of formula (I) or a pharmaceutically acceptable salt thereof in the composition is 0.05-10 %, preferably 0.1-2 %.

10. Non-therapeutic use of a compound of formula (I) or a pharmaceutically acceptable salt thereof: 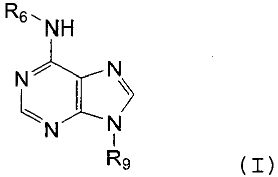 wherein R₆ is furfuryl, methoxy-substituted furfuryl, phenyl, methoxy-substituted phenyl or methoxy-substituted benzyl, and R₉ is 2-tetrahydropyranyl or 2-tetrahydrofuranyl, for improving the cosmetic appearance of mammalian epidermal cells.

11. The non-therapeutic use according to claim 10, wherein in formula (I) R₆ is furfuryl or methoxy-substituted furfuryl and R₉ is 2-tetrahydropyranyl.

12. The non-therapeutic use according to claim 11, wherein R₆ is furfuryl.

13. The non-therapeutic use according to any of claims 10-12, wherein the compound of formula (I) or a pharmaceutically acceptable salt thereof is present in a composition further comprising a cosmetic carrier.

14. The non-therapeutic use according to claim 13, wherein the content of the compound of formula (I) or a pharmaceutically acceptable salt thereof in the composition is 0.05-10 %, preferably 0.1-2 %.

## Patentansprüche

1. Verfahren zur Verbesserung des kosmetischen Aussehens von epidermalen Säugerzellen, umfassend die Verwendung einer Verbindung der Formel (I) oder eines pharmazeutisch akzeptablen Salzes davon: 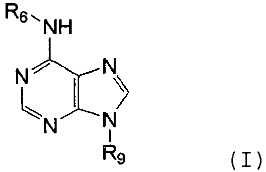 worin R₆ Furfuryl, Methoxy-substituiertes Furfuryl, Phenyl, Methoxy-substituiertes Phenyl oder Methoxy-substituiertes Benzyl ist und R₉ 2-Tetrahydropyranyl oder 2-Tetrahydrofuranyl ist.

2. Verfahren nach Anspruch 1, worin die Säugerzellen menschliche Hautzellen sind.

3. Verfahren nach Anspruch 1 oder 2, worin in der Formel (I) R₆ Furfuryl oder Methoxy-substituiertes Furfuryl und R₉ 2-Tetrahydropyranyl ist.

4. Verfahren nach Anspruch 3, worin R₆ Furfuryl ist.

5. Verfahren zur Verbesserung des kosmetischen Aussehens von menschlichen Hautzellen, umfassend die Verwendung einer Zusammensetzung, umfassend eine Verbindung der Formel (I) oder eines pharmazeutisch akzeptablen Salzes davon: 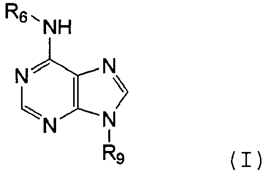 worin R₆ Furfuryl, Methoxy-substituiertes Furfuryl, Phenyl, Methoxy-substituiertes Phenyl oder Methoxy-substituiertes Benzyl ist und R₉ 2-Tetrahydropyranyl oder 2-Tetrahydrofuranyl ist.

6. Verfahren nach Anspruch 5, worin die menschlichen Hautzellen in einem lebenden Menschen sind.

7. Verfahren nach Anspruch 5 oder 6, worin in der Formel (I) R₆ Furfuryl oder Methoxy-substituiertes Furfuryl und R₉ 2-Tetrahydropyranyl ist.

8. Verfahren nach Anspruch 7, worin R₆ Furfuryl ist.

9. Verfahren nach einem der Ansprüche 5 bis 8, worin der Gehalt der Verbindung der Formel (I) oder eines pharmazeutisch akzeptablen Salzes davon in der Zusammensetzung 0,05-10 %, bevorzugt 0,1-2 % ist.

10. Nicht-therapeutische Verwendung einer Verbindung der Formel (I) oder eines pharmazeutisch akzeptablen Salzes davon: 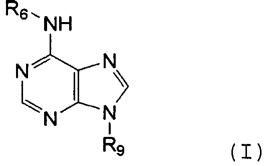 worin R₆ Furfuryl, Methoxy-substituiertes Furfuryl, Phenyl, Methoxy-substituiertes Phenyl oder Methoxy-substituiertes Benzyl ist und R₉ 2-Tetrahydropyranyl oder 2-Tetrahydrofuranyl ist, zur Verbesserung des kosmetischen Aussehens von epidermalen Säugerzellen.

11. Verfahren nach Anspruch 10, worin in der Formel (I) R₆ Furfuryl oder Methoxy-substituiertes Furfuryl und R₉ 2-Tetrahydropyranyl ist.

12. Verfahren nach Anspruch 11, worin R₆ Furfuryl ist.

13. Nicht-therapeutische Verwendung nach einem der Ansprüche 10 bis 12, worin die Verbindung der Formel (I) oder ein pharmazeutisch akzeptables Salz davon in einer Zusammensetzung vorhanden ist, die weiterhin einen kosmetischen Träger enthält.

14. Nicht-therapeutische Verwendung nach Anspruch 13, worin der Gehalt der Verbindung der Formel (I) oder eines pharmazeutisch akzeptablen Salzes davon in der Zusammensetzung 0,05-10 %, bevorzugt 0,1-2 % ist.

## Revendications

1. Procédé pour améliorer l'apparence cosmétique de cellules épidermiques de mammifère, comprenant l'utilisation d'un composé de formule (I) ou d'un sel pharmaceutiquement acceptable de celui-ci : 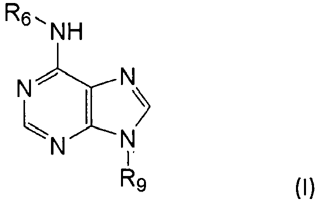 dans laquelle R₆ est furfuryle, furfuryle à substituant méthoxy, phényle, phényle à substituant méthoxy ou benzyle à substituant méthoxy, et Rg est 2-tétrahydropyranyle ou 2-tétrahydrofuranyle.

2. Le procédé selon la revendication 1, dans lequel les cellules de mammifère sont des cellules de peau humaine.

3. Le procédé selon la revendication 1 ou 2, dans lequel dans la formule (I) R₆ est furfuryle ou furfuryle à substituant méthoxy et Rg est 2-tétrahydropyranyle.

4. Le procédé selon la revendication 3, dans lequel R₆ est furfuryle.

5. Procédé pour améliorer l'apparence cosmétique de cellules de peau humaine, comprenant l'utilisation d'une composition comprenant un composé de formule (I) ou un sel pharmaceutiquement acceptable de celui-ci : 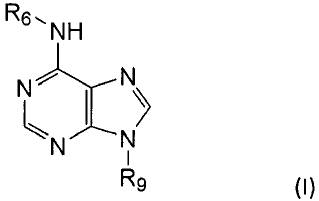 dans laquelle R₆ est furfuryle, furfuryle à substituant méthoxy, phényle, phényle à substituant méthoxy ou benzyle à substituant méthoxy, et Rg est 2-tétrahydropyranyle ou 2-tétrahydrofuranyle.

6. Le procédé selon la revendication 5, dans lequel les cellules de peau humaine sont dans un être humain vivant.

7. Le procédé selon la revendication 5 ou 6, dans lequel dans la formule (I) R₆ est furfuryle ou furfuryle à substituants méthoxy et Rg est 2-tétrahydropyranyle.

8. Le procédé selon la revendication 7, dans lequel R₆ est furfuryle.

9. Le procédé selon l'une quelconque des revendications 5-8, dans lequel la teneur du composé de formule (I) ou de son sel pharmaceutiquement acceptable de celui-ci dans la composition est de 0,05-10 %, de préférence de 0,1-2 %.

10. Utilisation non thérapeutique d'un composé de formule (I) ou d'un sel pharmaceutiquement acceptable de celui-ci : 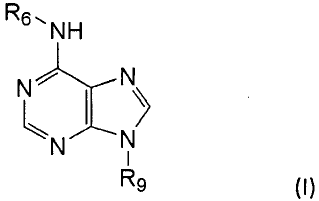 dans laquelle R₆ est furfuryle, furfuryle à substituant méthoxy, phényle, phényle à substituant méthoxy ou benzyle à substituant méthoxy, et Rg est 2-tétrahydropyranyle ou 2-tétrahydrofuranyle, pour améliorer l'apparence cosmétique de cellules épidermiques de mammifère.

11. L'utilisation non-thérapeutique selon la revendication 10, dans laquelle dans la formule (I) R₆ est furfuryle ou furfuryle à substituant méthoxy et Rg est 2-tétrahydropyranyle.

12. L'utilisation non-thérapeutique selon la revendication 11, dans laquelle R₆ est furfuryle.

13. L'utilisation non-thérapeutique selon l'une quelconque des revendications 10-12, dans laquelle le composé de formule (I) ou un sel pharmaceutiquement acceptable de celui-ci est présent dans une composition comprenant en outre un support cosmétique.

14. L'utilisation non-thérapeutique selon la revendication 13, dans laquelle la teneur du composé de formule (I) ou de son sel pharmaceutiquement acceptable de celui-ci dans la composition est de 0,05-10 %, de préférence de 0,1-2 %.
